(19) Europäisches Patentamt
European Patent Office
Office européen des brevets

(11) Veröffentlichungsnummer: **0 379 928 A2**

(12) **EUROPÄISCHE PATENTANMELDUNG**

(21) Anmeldenummer: **90100786.4**

(22) Anmeldetag: **16.01.90**

(51) Int. Cl.[5]: **C07D 213/36, C07D 213/61, C07D 213/69, C07D 213/64, C07D 213/00, C07D 213/40, C07D 213/28, A61K 31/44, A23K 1/16**

(30) Priorität: **26.01.89 DE 3902286**
**09.06.89 DE 3918834**

(43) Veröffentlichungstag der Anmeldung:
**01.08.90 Patentblatt 90/31**

(84) Benannte Vertragsstaaten:
**AT BE CH DE DK ES FR GB GR IT LI NL SE**

(71) Anmelder: **BAYER AG**

**D-5090 Leverkusen 1 Bayerwerk(DE)**

(72) Erfinder: **Lindel, Hans, Dr.**
**Carl-Duisberg-Strasse 321**
**D-5090 Leverkusen 1(DE)**
Erfinder: **Hallenbach, Werner, Dr.**
**Lichtenberger Strasse 68**
**D-4019 Monheim(DE)**
Erfinder: **Berschauer, Friedrich, Prof. Dr.**
**Bauermannskulle 66**
**D-5650 Solingen 1(DE)**
Erfinder: **Greife, Heinrich A., Prof. Dr.**
**Salmstrasse 23**
**D-4018 Langenfeld(DE)**
Erfinder: **Klotz, Gernot, Dr.**
**Foerster-Sons-Strasse 32**
**D-5653 Leichlingen(DE)**

(54) **Aryl- und Heteroarylethanol-pyridylalkylamine, Verfahren zu ihrer Herstellung und ihre Verwendung als Leistungsförderer bei Tieren und als Mittel gegen Adipositas.**

(57) Neue Aryl- und Heteroarylethanol-pyridylalkylamine der Formel I

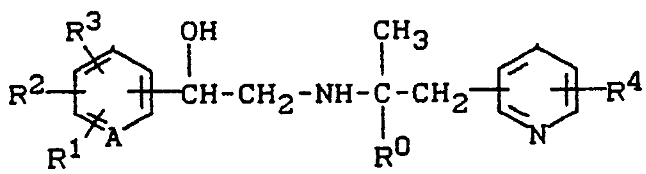

(I)

in welcher
A für =CH- oder =N- steht,
$R^0$ für Wasserstoff oder Methyl steht,
$R^1$ für Wasserstoff, Hydroxy, Halogen, Cyano, Alkyl, Halogenalkyl, Hydroxyalkyl, Alkoxycarbonyl, Aminocarbonyl, Mono- und Dialkylaminocarbonyl, Alkoxy, Halogenalkoxy, Halogenalkylthio, $NHSO_2$-Alkyl steht,
$R^2$ für Wasserstoff, Hydroxy, Alkoxy oder den Rest $-NR^5R^6$ steht,
$R^3$ für die bei $R^1$ angegebenen Reste steht,
$R^4$ für Wasserstoff, $C_1$-$C_{10}$-Alkyl, welches gegebenenfalls durch Hydroxy, Halogen, Alkoxy, Acyloxy oder den Rest $-NR^7R^8$ substituiert ist, sowie für den Rest $COR^9$ oder den Rest $-O-Z-R^{10}$ steht,
Z für $C_1$-$C_{10}$-Alkylen, -Alkenylen oder -Alkinylen steht,
$R^5$ für Wasserstoff oder Alkyl steht,
$R^6$ für Wasserstoff, Alkyl, Halogenalkyl oder Acyl steht, wobei $R^5$ und $R^6$ gemeinsam mit dem angrenzen-

EP 0 379 928 A2

Xerox Copy Centre

den N-Atom einen gesättigten oder ungesättigten heterocyclischen 4-, 5- oder 6-Ring bilden können,
$R^7$ für Wasserstoff, gegebenenfalls substituiertes Alkyl, gegebenenfalls substituiertes Aryl steht,
$R^8$ für die bei $R^7$ angegebenen Reste steht,
$R^9$ für Hydroxy, Alkoxy oder den Rest $-NR^7R^8$ steht,
$R^{10}$ für Hydroxy, Alkoxy, Acyloxy, gegebenenfalls substituiertes Aryloxy oder Aralkyloxy steht,
wobei im Pyridylring der Formel I der Substituent $R^4$ und die Alkylaminogruppe in p-Stellung zueinander stehen
sowie ihre physiologisch verträglichen Salze und, wenn A für Stickstoff steht, ihre N-Oxide.

Die vorliegende Erfindung betrifft ferner Verfahren zur Herstellung der Verbindungen, dabei verwendete Zwischenprodukte und ihre Verwendung als Leistungsförderer bei Tieren sowie als Mittel gegen Adipositas.

# Aryl- und Heteroarylethanol-pyridylalkylamine, Verfahren zu ihrer Herstellung und ihre Verwendung als Leistungsförderer bei Tieren und als Mittel gegen Adipositas

Die vorliegende Erfindung betrifft neue Aryl- und Heteroarylethanol-pyridylalkylamine, Verfahren zur ihrer Herstellung und ihre Verwendung als Leistungsförderer bei Tieren sowie als Mittel zur Behandlung der Adipositas bei Mensch und Tier.

Aryl- und Heteroarylethanolamine sind bereits bekannt. Sie eignen sich als Leistungsförderer für Tiere. Ihre Wirkung ist jedoch nicht in jedem Fall befriedigend. (EP-OS 26 298; EP-OS 25 856; EP-OS 170 538; EP-OS 256 420)

Es wurde nun gefunden:

1. Neue Aryl- und Heteroarylethanol-pyridylalkylamine der Formel I

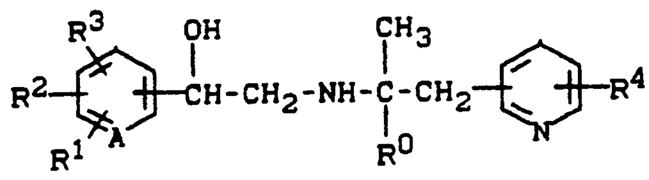

(I)

in welcher
A für =CH- oder =N- steht,
$R^0$ für Wasserstoff oder Methyl steht,
$R^1$ für Wasserstoff, Hydroxy, Halogen, Cyano, Alkyl, Halogenalkyl, Hydroxyalkyl, Alkoxycarbonyl, Aminocarbonyl, Mono- und Dialkylaminocarbonyl, Alkoxy, Halogenalkoxy, Halogenalkylthio, $NHSO_2$-Alkyl steht,
$R^2$ für Wasserstoff, Hydroxy, Alkoxy oder den Rest $-NR^5R^6$ steht,
$R^3$ für die bei $R^1$ angegebenen Reste steht,
$R^4$ für Wasserstoff, $C_1$-$C_{10}$-Alkyl, welches gegebenenfalls durch Hydroxy, Halogen, Alkoxy, Acyloxy oder den Rest $-NR^7R^8$ substituiert ist, sowie für den Rest $COR^9$ oder den Rest $-O-Z-R^{10}$ steht,
Z für $C_1$-$C_{10}$-Alkylen, -Alkenylen oder -Alkinylen steht,
$R^5$ für Wasserstoff oder Alkyl steht,
$R^6$ für Wasserstoff, Alkyl, Halogenalkyl oder Acyl steht, wobei $R^5$ und $R^6$ gemeinsam mit dem angrenzenden N-Atom einen gesättigten oder ungesättigten heterocyclischen 4-, 5- oder 6-Ring bilden können,
$R^7$ für Wasserstoff, gegebenenfalls substituiertes Alkyl, gegebenenfalls substituiertes Aryl steht,
$R^8$ für die bei $R^7$ angegebenen Reste steht,
$R^9$ für Hydroxy, Alkoxy oder den Rest $-NR^7R^8$ steht,
$R^{10}$ für Hydroxy, Alkoxy, Acyloxy, gegebenenfalls substituiertes Aryloxy oder Aralkyloxy steht,
wobei im Pyridylring der Formel I der Substituent $R^4$ und die Alkylaminogruppe in p-Stellung zueinander stehen
sowie ihre physiologisch verträglichen Salze und, wenn A für Stickstoff steht, ihre N-Oxide.

2. Verfahren zur Herstellung der Verbindungen der Formel I

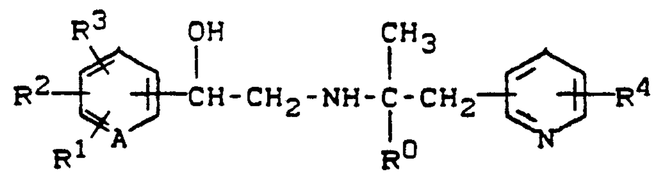

(I)

in welcher
A für =CH- oder =N- steht,
$R^0$ für Wasserstoff oder Methyl steht,
$R^1$ für Wasserstoff, Alkyl, Halogen, Halogenalkyl, Hydroxy, Hydroxyalkyl, Cyano, Alkoxycarbonyl, Aminocarbonyl, Mono- und Dialkylaminocarbonyl, Alkoxy, Halogenalkoxy, Halogenalkylthio, $NHSO_2$-Alkyl steht,
$R^2$ für Wasserstoff, Hydroxy, Alkoxy oder den Rest-$NR^5R^6$ steht,
$R^3$ für die bei $R^1$ angegebenen Reste steht,
$R^4$ für Wasserstoff, $C_1$-$C_{10}$-Alkyl, welches gegebenenfalls durch Hydroxy, Halogen, Alkoxy, Acyloxy oder den Rest $-NR^7R^8$ substituiert ist, sowie für den Rest $COR^9$ oder den Rest $-O-Z-R^{10}$ steht,

Z für $C_1$-$C_{10}$-Alkylen, -Alkenylen oder -Alkinylen steht,
$R^5$ für Wasserstoff oder Alkyl steht,
$R^6$ für Wasserstoff, Alkyl, Halogenalkyl oder Acyl steht, wobei $R^5$ und $R^6$ gemeinsam mit dem angrenzenden N-Atom einen gesättigten oder ungesättigten heterocyclischen 4-,5- oder 6-Ring bilden können,
$R^7$ für Wasserstoff, gegebenenfalls substituiertes Alkyl, gegebenenfalls substituiertes Aryl steht,
$R^8$ für die bei $R^7$ angegebenen Reste steht,
$R^9$ für Hydroxy, Alkoxy oder den Rest $-NR^7R^8$ steht,
$R^{10}$ für Hydroxy, Alkoxy, Acyloxy, gegebenenfalls substituiertes Aryloxy oder Aralkyloxy steht,
wobei im Pyridylring der Formel I der Substituent $R^4$ und die Alkylaminogruppe in p-Stellung zueinander stehen.

a) indem man Halogenmethylketone der Formel II

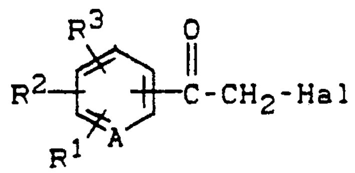
(II)

in welcher
$R^1$ bis $R^3$ und A die oben angegebene Bedeutung haben und Hal für Halogen steht,
mit Aminen der Formel (III)

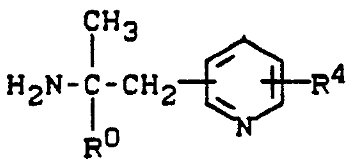
(III)

in welcher
$R^0$ und $R^4$ die oben angegebene Bedeutung haben und $R^4$ in p-Stellung zum Alkylaminrest steht,
umsetzt und anschließend die Carbonylgruppe reduziert, oder

b) indem man Epoxide der Formel IV

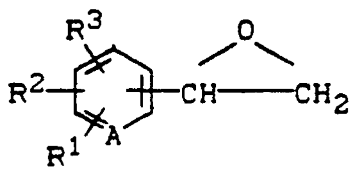
(IV)

in welcher
$R^1$ bis $R^3$ und A die oben angegebene Bedeutung haben,
mit Aminen der Formel III

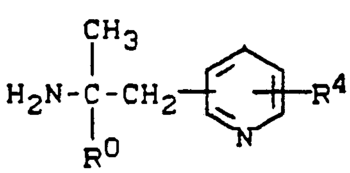
(III)

in welcher
$R^0$ und $R^4$ die oben angegebene Bedeutung haben und $R^4$ in p-Stellung zum Alkylaminrest steht,
umsetzt, oder

c) indem man ß-Halogenethylverbindungen der Formel V

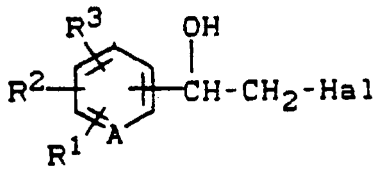

(V)

in welcher
$R^1$ bis $R^3$ und A die oben angegebene Bedeutung haben und Hal für Halogen steht, mit Aminen der Formel III

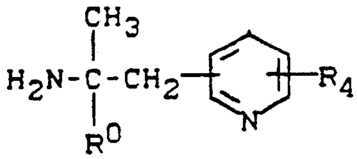

(III)

in welcher
$R^0$ und $R^4$ die oben angegebene Bedeutung haben und $R^4$ in p-Stellung zum Alkylaminrest steht, umsetzt, oder

d) indem man für den Fall, daß $R^0$ für Wasserstoff steht, Verbindungen der Formel VI

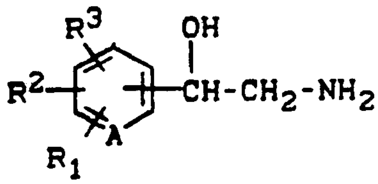

(VI)

in welcher
$R^1$ bis $R^3$ und A die angegebene Bedeutung haben, mit Ketonen der Formel VII

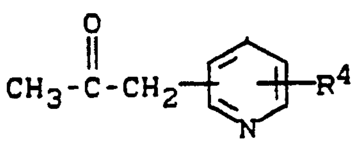

(VII)

in welcher
$R^4$ die oben angegebene Bedeutung hat und in p-Stellung zum Acetonylrest steht, unter reduzierenden Bedingungen umsetzt, oder

e) indem man Verbindungen der Formel VIII

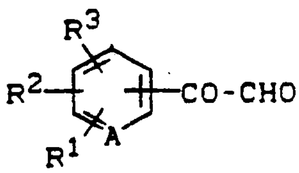

(VIII)

in welcher
$R^1$ bis $R^3$ und A die oben angegebenen Bedeutung haben, mit Aminen der Formel III

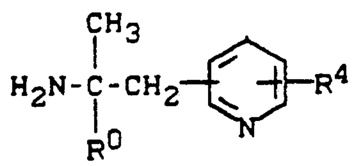

(III)

in welcher
$R^0$ und $R^4$ die oben angebenene Bedeutung haben und $R^4$ in p-Stellung zum Alkylaminrest steht, unter reduzierenden Bedingungen umsetzt, oder

f) indem man Verbindungen der Formel IX

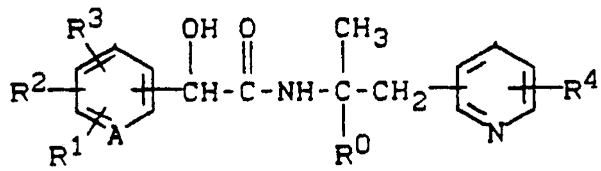

(IX)

in welcher
A und $R^0$ bis $R^4$ die oben angegebene Bedeutung haben, und $R^4$ und der andere Substituent im Pyridinring der Formel IX in p-Stellung zueinander stehen,
reduziert.

3. Neue Verbindungen der Formel III

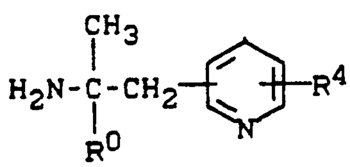

(III)

in welcher
$R^0$ und $R^4$ die bei den Verbindungen der Formel I angegebenen Bedeutungen haben, $R^4$ aber nicht für Wasserstoff steht, und die Substituenten am Pyridinring in p-Position zueinander stehen.

4. Verfahren zur Herstellung der Verbindungen der Formel III gemäß 3, dadurch gekennzeichnet, daß man

a) für den Fall, daß $R^0$ für Wasserstoff steht, Verbindungen der Formel VII

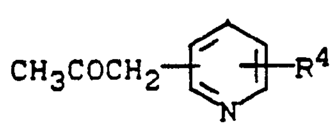

(VII)

in welcher
$R^4$ die bei den Verbindungen der Formel I angegebenen Bedeutungen hat und die Substituenten am Pyridinring der Formel (VII) in p-Position zueinander stehen

a) in Gegenwart von Ammoniak reduziert oder

b) in ihr Oxim, ihren Oximether oder -ester überführt und diese anschließend reduziert, oder daß man

b) für den Fall, daß $R^0$ für Methyl steht, Verbindungen der Formel (XXII)

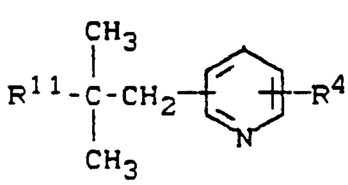

(XXII)

in welcher
$R^4$ die oben angegebene Bedeutung hat,
$R^{11}$ für die Reste $-NH-COCH_3$ oder -NH-CHO steht
mit Basen umsetzt.

5. Neue Verbindungen der Formel IX

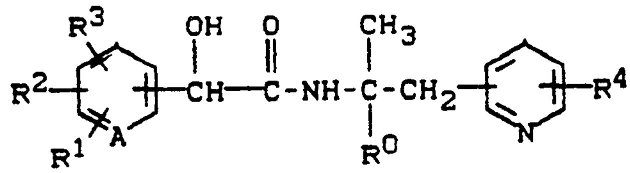

(IX)

in welcher
A und $R^0$ bis $R^4$ die bei den Verbindungen der Formel I angegebenen Bedeutungen haben, wobei $R^4$ und die Alkylaminseitenkette im Pyridinring der Formel (IX) in p-Position zueinander stehen.

6. Verfahren zur Herstellung der Verbindungen der Formel IX gemäß 5, dadurch gekennzeichnet, daß man Verbindungen der Formel X

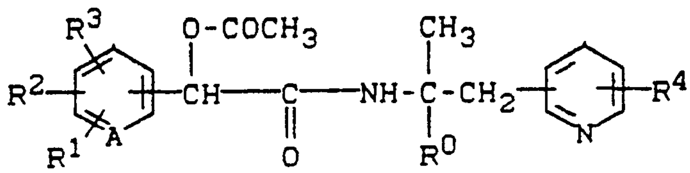

(X)

in welcher
A und $R^0$ bis $R^4$ die oben angegebene Bedeutung haben und $R^4$ und die Alkylaminseitenkette im Pyridinring in p-Position zueinander stehen,
hydrolysiert.

7. Neue Verbindungen der Formel X

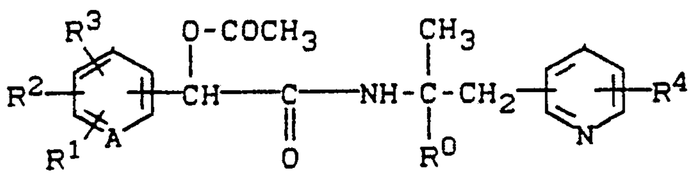

(X)

in welcher
$R^0$ bis $R^4$ und A die oben angegebene Bedeutung haben und $R^4$ und die Alkylaminseitenkette im Pyridinring in p-Position zueinander stehen.

8. Verfahren zur Herstellung der Verbindungen der Formel X gemäß 7, dadurch gekennzeichnet, daß man Aldehyde der Formel XI

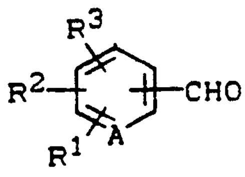

(XI)

in welcher
$R^1$ bis $R^3$ und A die oben angegebene Bedeutung haben,
mit Isonitrilen der Formel XII

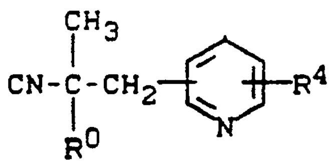

(XII)

in welcher
$R^0$ und $R^4$ die oben angegebenen Bedeutungen hat und die Substituenten am Pyridinring de Formel (XII) in p-Position zueinander stehen,
in Gegenwart von Essigsäure umsetzt.

9. Neue Verbindungen der Formel XII

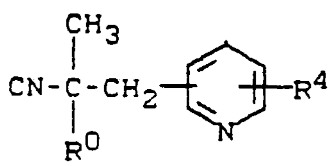

(XII)

in welcher
$R^0$ und $R^4$ die oben angegebene Bedeutung haben und die Substituenten am Pyridinring der Formel (XII) in p-Position zueinander stehen.

10. Verfahren zur Herstellung der Verbindungen der Formel XII gemäß 9, dadurch gekennzeichnet, daß man Amine der Formel III

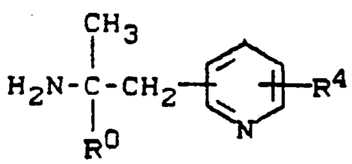

(III)

in welcher
$R^0$ und $R^4$ die oben angegebene Bedeutung haben und die Substituenten am Pyridinring der Formel (III) in p-Position zueinander stehen, in an sich bekannter Weise zunächst in die Formylverbindung überführt und diese dann mit Phosgen oder Phosphoroxychlorid umsetzt.

11. Neue Verbindungen der Formel XIII

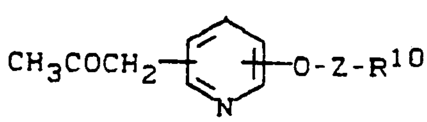

(XIII)

in welcher
$R^{10}$ und Z die bei (1) oben angegebene Bedeutung haben und die Substituenten am Pyridinring der Formel (XIII) in p-Position zueinander stehen.

12. Verfahren zur Herstellung der Verbindungen der Formel XIII gemäß 11, dadurch gekennzeichnet, daß man

a) für den Fall, daß die Acetonylgruppe in 2-Stellung und der Rest -O-Z-$R^{10}$ in 5-Stellung stehen, Verbindungen der Formel XIV

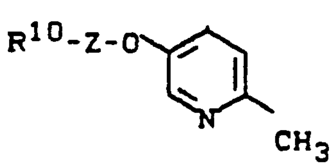

(XIV)

in welcher
$R^{10}$ und Z die oben angegebene Bedeutung haben,
lithiiert und anschließend mit N,N-Dimethylacetamid umsetzt,

b) für den Fall, daß die Acetonylgruppe in 3-Stellung und der Rest -O-Z-$R^{10}$ in 6-Stellung stehen, Verbindungen der Formel XV

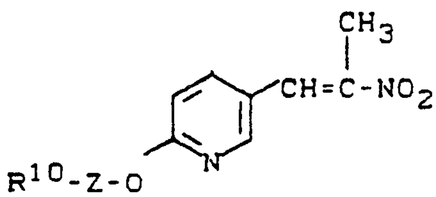

(XV)

in welcher
$R^{10}$ und Z die oben angegebene Bedeutung haben, reduziert und hydrolysiert.

13. Neue Verbindungen der Formel XVI

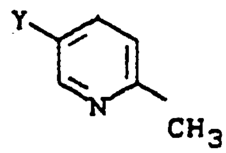

(XVI)

in welcher
Y für -O-Z-$R^{10}$, ausgenommen 2-Methoxyethoxy oder 2-Ethoxyethoxy, steht.

14. Verfahren zur Herstellung der Verbindungen der Formel XVI gemäß 13, dadurch gekennzeichnet, daß man 2-Methyl-5-hydroxypyridin mit einer Verbindung der Formel XVII

$R^{10}$-Z-X (XVII)

in welcher
$R^{10}$ und Z die oben angegebene Bedeutung haben und
X für Halogen, Mesylat oder Tosylat steht,
alkyliert.

15. Neue Verbindungen der Formel XV

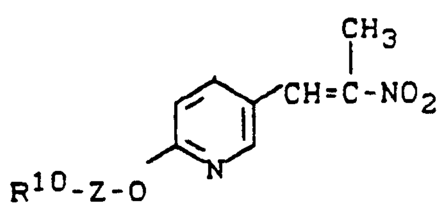

(XV)

in welcher
$R^{10}$ und Z die oben angegebenen Bedeutungen haben,
wobei
$R^{10}$-Z- nicht für Methyl steht.

16. Verfahren zur Herstellung der Verbindungen der Formel XV gemäß 15, dadurch gekennzeichnet, daß man Aldehyde der Formel XVIII

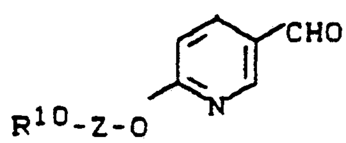

(XVIII)

in welcher
$R^{10}$ und Z die oben angegebene Bedeutung haben, mit Nitroethan kondensiert.

17. Neue Verbindungen der Formel XVIII

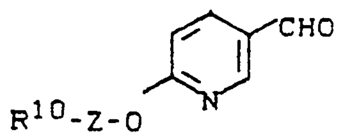

(XVIII)

in welcher
$R^{10}$ und Z die oben angegebene Bedeutung haben, wobei
$R^{10}$-Z- nicht für Methyl steht.

18. Verfahren zur Herstellung der Verbindungen der Formel XVIII gemäß 17, dadurch gekennzeichnet, daß man

a) Alkohole der Formel XIX

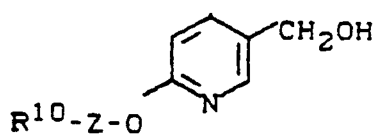

(XIX)

in welcher
$R^{10}$ und Z die unter 17 angegebene Bedeutung haben, oxidiert oder

b) eine Verbindung der Formel XXVI

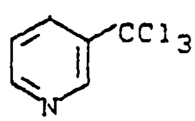

(XXVI)

mit einer Verbindung der Formel
$R^{10}$-Z-OH (XXI)
in welcher $R^{10}$ und Z die oben angegebene Bedeutung haben,
im Gegenwart von Basen umsetzt.

19. Neue Verbindungen der Formel XIX

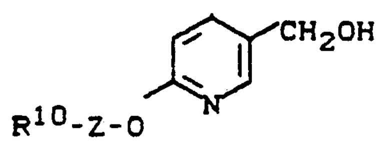

(XIX)

in welcher
$R^{10}$ und Z die oben angegebene Bedeutung haben, und $R^{10}$-Z- nicht für Methyl steht.

20. Verfahren zur Herstellung der Verbindungen der Formel XIX gemäß 19, dadurch gekennzeichnet, daß man Carbonsäuren der Formel XX

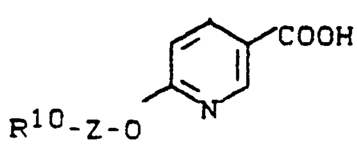

(XX)

in welcher
$R^{10}$ und Z die oben angegebene Bedeutung haben,
reduziert.

21. Neue Verbindungen der Formel XX

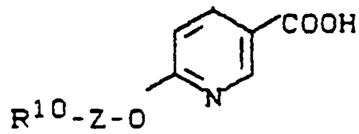

(XX)

in welcher
$R^{10}$ und Z die oben angegebene Bedeutung haben,
wobei
$R^{10}$-Z- nicht für Methyl steht.

22. Verfahren zur Herstellung der Verbindungen der Formel XX gemäß 21, dadurch gekennzeichnet, daß man 6-Chlornicotinsäure mit einer Verbindung der Formel XXI
$R^{10}$-Z-OH (XXI)
wobei
$R^{10}$ und Z die oben angegebene Bedeutung haben,
umsetzt.

23. Neue Verbindungen der Formel (XXII)

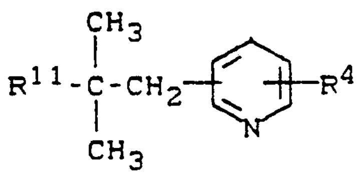

(XXII)

in welcher
$R^4$ die bei den Verbindungen der Formel (I) angegebenen Bedeutungen hat und die Substituenten am Pyridinring der Formel (XXII) in p-Position zueinander stehen,
$R^{11}$ für die Reste -NHCHO und
$NH-\overset{O}{\overset{\|}{C}}-CH_3$ steht.

24. Verfahren zur Herstellung der Verbindungen der Formel (XXII) gemäß 23, dadurch gekennzeichnet, daß man vinylsubstituierte Pyridine der Formel (XXIII)

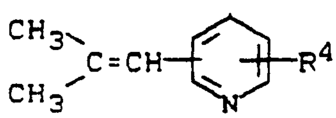

(XXIII)

in welcher
$R^4$ die bei den Verbindungen der Formel (I) angegegebene Bedeutung hat und die Substituenten am Pyridinring der Formel (XXIII) in p-Position zu einander stehen,
mit organischen Nitrilen, Alkalicyaniden oder Blausäure in Gegenwart organischer oder anorganischer Säuren umsetzt.

25. Neue Verbindungen der Formel (XXIII)

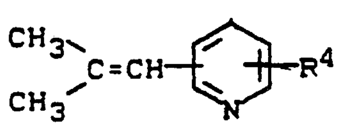

(XXIII)

in welcher
$R^4$ die bei den Verbindungen der Formel (I) angegegebene Bedeutung hat und die Substituenten am Pyridinring der Formel (XXIII) in p-Position zueinander stehen.

26. Verfahren zur Herstellung der Verbindungen der Formel (XXIII) gemäß 25, dadurch gekennzeichnet, daß man Pyridine der Formel (XXIV)

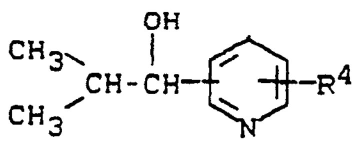

(XXIV)

in welcher
$R^4$ die bei den Verbindungen der Formel (I) angegegebene Bedeutung hat und die Substituenten am Pyridinring der Formel (XXIV) in p-Position zu einander stehen,

a) in Gegenwart von Protonensäuren umsetzt oder

b) zunächst mit einem Halogenierungsmittel und anschließend mit einer Base umsetzt.

27. Neue Verbindungen der Formel (XXIV)

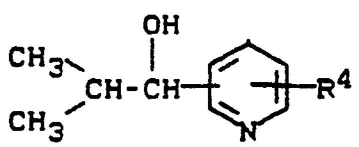

(XXIV)

in welcher
$R^4$ die bei den Verbindungen der Formel (I) angegegebene Bedeutung hat und die Substituenten am Pyridinring der Formel (XXIV) in p-Position zu einander stehen.

28. Verfahren zur Herstellung der Verbindungen der Formel (XXIV) gemäß 27, dadurch gekennzeichnet, daß man Verbindungen der Formel (XVIII)

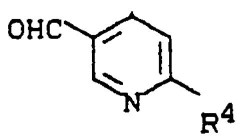

(XVIII)

in welcher
$R^4$ die bei den Verbindungen der Formel (I) angegegebene Bedeutung hat,
mit Grignard-Verbindungen der Formel (XXV)

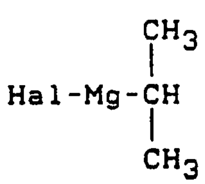

(XXV)

in welcher
Hal für Halogen steht,
umsetzt.

Die Verbindungen der Formel I können, wenn A für Stickstoff steht, in Form ihrer Tautomeren vorliegen. Beispiele dafür sind für den Fall, daß $R^2$ für $NH_2$ steht:

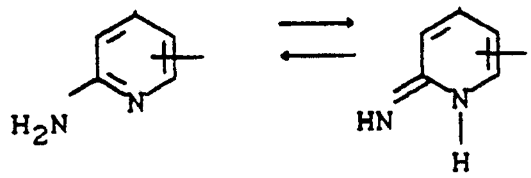

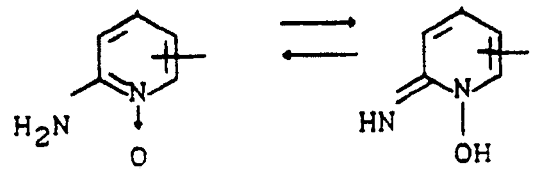

Die Verbindungen der Formel I können auch in Form ihrer sterischen und optischen Isomeren vorliegen und dabeizueinander enantiomere und/oder diastereomere Formen bilden.

Physiologisch verträgliche Salze der Verbindungen der Formel I könen mit folgenden Säuren gebildet werden:

Salzsäure, Schwefelsäure, Phosphorsäure, Perchlorsäure, Brom-, Iodwasserstoffsäure, Salpetersäure, Essigsäure, Oxalsäure, Malonsäure, Bernsteinsäure, Ascorbinsäure, Äpfelsäure, Weinsäure, Maleinsäure, Fumarsäure, Methansulfonsäure, Benzoesäure, substituierte Benzoesäuren, Ameisensäure, Toluolsulfonsäure, Benzolsulfonsäure, Phthalsäure, Naphthalinsulfonsäure, Nikotinsäure, Palmitinsäure, Embonsäure.

Bevorzugt sind Verbindungen der Formel I,
in welcher
A für =CH- oder =N- steht,
$R^0$ für Wasserstoff oder Methyl steht,
$R^1$ für Wasserstoff, Hydroxy, Fluor, Chlor, Brom, Cyano, $C_1$-$C_4$-Alkyl, $C_1$-$C_4$-Halogenalkyl, Hydroxymethyl, $C_1$-$C_4$-Alkoxycarbonyl, Aminocarbonyl, Mono-oder Di-$C_1$-$C_4$-alkylaminocarbonyl, $C_1$-$C_6$-Alkoxy, $C_1$-$C_6$-Halogenalkoxy, $C_1$-$C_6$-Halogenalkylthio, $NHSO_2$-$C_1$-$C_6$-Alkyl steht,
$R^2$ für Wasserstoff, Hydroxy, Alkoxy oder den Rest $-NR^5R^6$ steht,
$R^3$ für die bei $R^1$ angebenen Reste steht,
$R^4$ für Wasserstoff, $C_1$-$C_6$-Alkyl, welches gegebenenfalls durch Hydroxy, $C_1$-$C_6$-Alkoxy, $C_1$-$C_6$-Acyloxy oder den Rest $-NR^7R^8$ substituiert ist, sowie für den Rest $COR^9$ oder den Rest $-O-Z-R^{10}$ steht,
Z für $C_1$-$C_6$-Alkylen, $C_2$-$C_6$-Alkenylen oder $C_2$-$C_6$-Alkinylen steht,
$R^5$ für Wasserstoff oder $C_1$-$C_6$-Alkyl steht,
$R^6$ für Wasserstoff, $C_1$-$C_6$-Alkyl, $C_1$-$C_6$-Halogenalkyl $C_1$-$C_6$-Alkylcarbonyl, gegebenenfalls substituiertes Phenylsulfonyl steht,
$R^7$ für Wasserstoff, $C_1$-$C_6$-Alkyl, welches gegebenenfalls substituiert ist, steht,
$R^8$ für die bei $R^7$ angegebenen Reste steht,
$R^9$ für Hydroxy, $C_1$-$C_6$-Alkoxy oder den Rest $-NR^7R^8$ steht,
$R^{10}$ für Hydroxy, $C_1$-$C_6$-Alkoxy, $C_1$-$C_6$-Acyloxy, gegebenenfalls substituiertes Aryloxy oder Aralkyloxy steht,
wobei $R^4$ und die Alkylaminogruppe am Pyridinring der Formel I in p-Stellung zueinander stehen.

Als Substituenten der gegebenenfalls substituierten Reste kommen bevorzugt infrage: Cyano, Halogen wie Fluor oder Chlor, Hydroxy, $C_{1-4}$-Alkyl, $C_{1-4}$-Halogenalkyl, Phenyl, $C_{1-4}$-Alkoxy, $C_{1-4}$-Halogenalkoxy, $C_{1-4}$-Alkylthio, $C_{1-4}$-Halogenalkylthio, für den Fall, daß die Substituenten an einem Phenylrest sitzen, zusätzlich bevorzugt Methylendioxy, Ethylendioxy, halogensubstituiertes Methylendioxy, halogensubstituiertes Ethylendioxy, ferner Phenyl, Phenoxy, die ihrerseits einen oder mehrere der obengenannten Substituenten tragen können.

Besonders bevorzugt sind Verbindungen der Formel I,
in welcher
A für =CH- oder =N- steht,
$R^0$ für Wasserstoff oder Methyl steht,
$R^1$ für Wasserstoff, Methyl, Ethyl, Fluor, Chlor, Brom, Hydroxy, Hydroxymethyl, Cyano, Methoxy- und Ethoxycarbonyl, Aminocarbonyl, Mono- oder Di-$C_1$-$C_4$-alkylaminocarbonyl, $C_1$-$C_4$-Alkoxy, $C_1$-$C_4$-Halogenalkoxy mit 1 bis 5 Halogenatomen, $-NHSO_2$-$C_1$-$C_4$-Alkyl steht,
$R^2$ für Wasserstoff, Hydroxy, $C_1$-$C_4$-Alkoxy oder den Rest $-NR^5R^6$ steht,
$R^3$ für die bei $R^1$ angegebenen Reste steht,
$R^4$ für Wasserstoff, $C_1$-$C_3$-Alkyl, welches bevorzugt durch Hydroxy, $C_1$-$C_3$-Alkoxy, insbesondere Methoxy, $C_1$-$C_3$-Acyloxy, insbesondere Acetoxy oder den Rest $-NR^7R^8$ substituiert ist, sowie für den Rest $COR^9$ oder

den Rest -O-Z-$R^{10}$ steht,
Z für $C_1$-$C_4$-Alkylen, $C_2$-$C_4$-Alkenylen oder $C_2$-$C_4$-Alkinylen steht,
$R^5$ für Wasserstoff oder $C_1$-$C_3$-Alkyl, insbesondere Methyl oder Ethyl steht,
$R^6$ für Wasserstoff, Methyl oder Acetyl steht,
$R^7$ für Wasserstoff, $C_1$-$C_3$-Alkyl, insbesondere Methyl oder Ethyl steht,
$R^8$ für Wasserstoff steht,
$R^9$ für Hydroxy, $C_1$-$C_3$-Alkoxy, insbesondere Methoxy oder Ethoxy, oder den Rest -$NR^7R^8$ steht,
$R^{10}$ für Hydroxy, $C_1$-$C_3$-Alkoxy, insbesondere Methoxy oder Ethoxy, $C_1$-$C_3$-Acyloxy, insbesondere Acetoxy, gegebenenfalls substituiertes Aryloxy oder Aralkyloxy steht,
wobei $R^4$ und die Alkylaminogruppe am Pyridylring in p-Stellung zueinander stehen.

Ganz besonders bevorzugt sind Verbindungen der folgenden Struktur

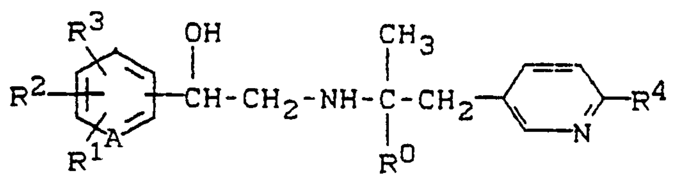 ,

in welcher
A für =CH- oder =N- steht,
$R^0$ bis $R^3$ die weiter oben angegebenen Bedeutungen oder bevorzugten Bedeutungen besitzen.
$R^4$ für den Rest -O-Z-$R^{10}$ steht, wobei Z und $R^{10}$ die weiter oben angebenen bevorzugten Bedeutungen besitzen.

Im einzelnen seien neben den Beispielen die folgenden Verbindungen der Formel I genannt:

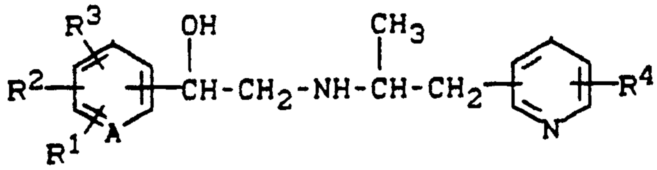 (I)

| 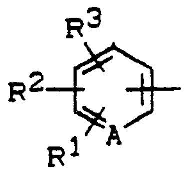 | 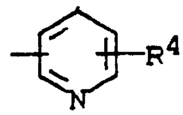 |
| --- | --- |
| 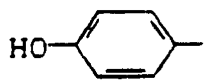 | 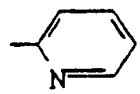 |
| 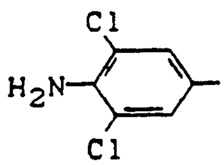 | 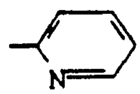 |
| 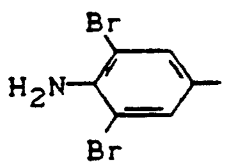 | 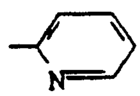 |
| 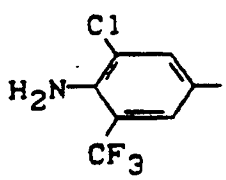 | 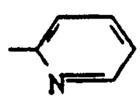 |
| 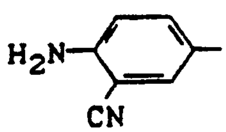 | 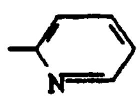 |

| 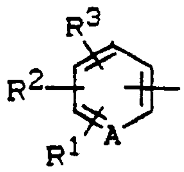 | 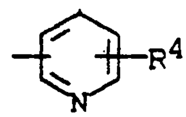 |
|---|---|
| 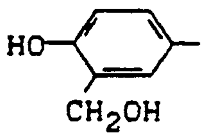 | 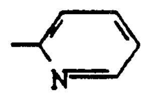 |
| 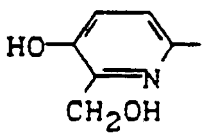 | 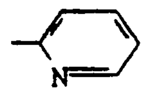 |
| 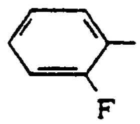 | 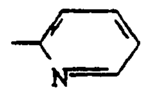 |
| 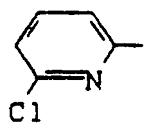 | 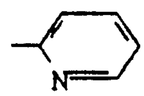 |
| 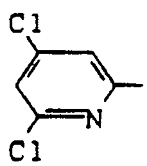 | 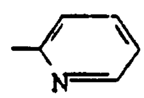 |

| 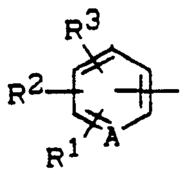 | 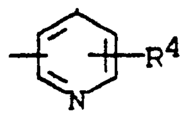 |
| --- | --- |
| 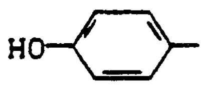 | 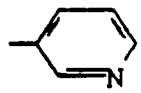 |
| 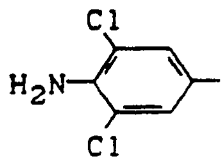 | 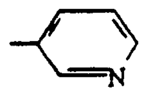 |
| 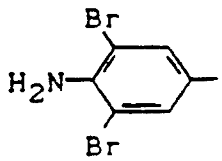 | 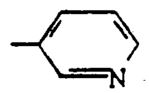 |
| 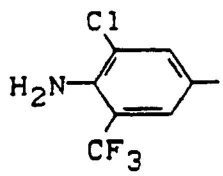 | 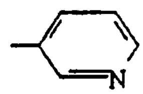 |
| 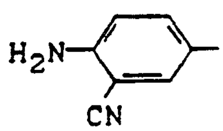 | 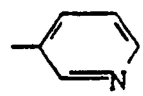 |
| 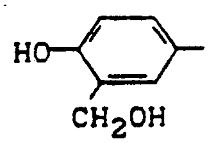 | 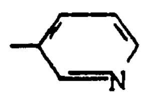 |
| 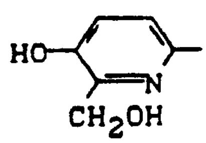 | 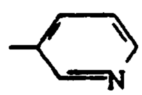 |

| 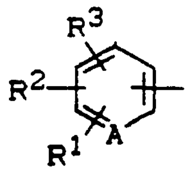 | 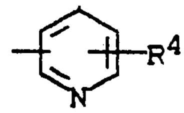 |
|---|---|
| 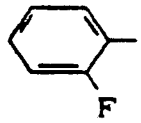 | 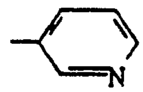 |
| 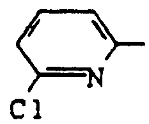 | 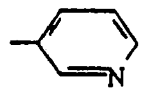 |
| 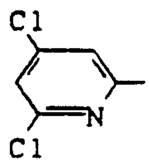 | 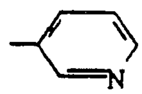 |
| 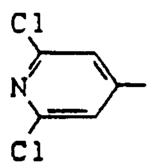 | 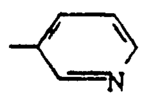 |

| 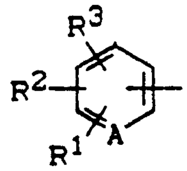 | 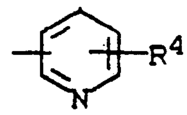 |
|---|---|
| 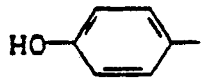 | 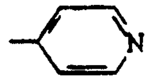 |
| 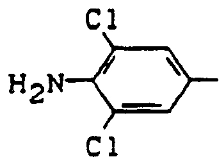 | 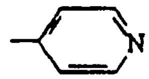 |
| 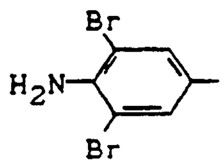 | 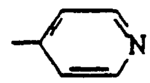 |
| 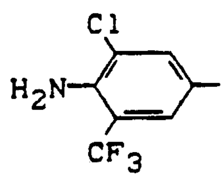 | 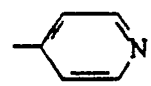 |
| 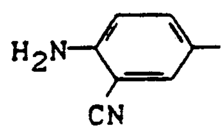 | 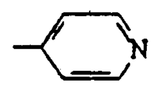 |
| 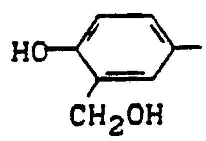 | 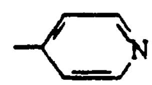 |
| 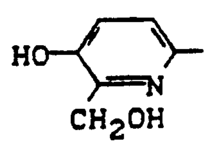 | 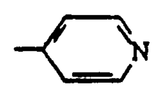 |

| 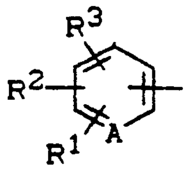 | 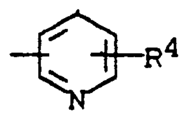 |
| --- | --- |
| 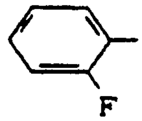 | 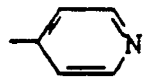 |
| 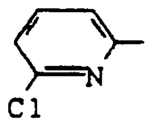 | 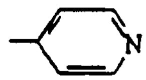 |
| 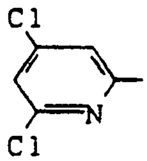 | 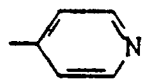 |
| 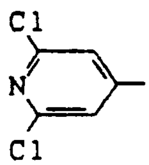 | 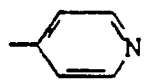 |

| 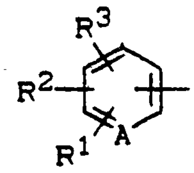 | 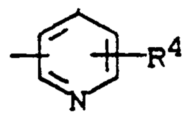 |
|---|---|
| 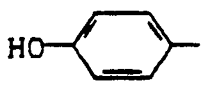 | 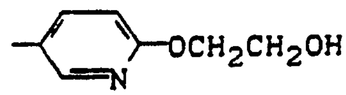 |
| 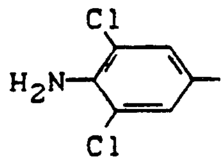 | 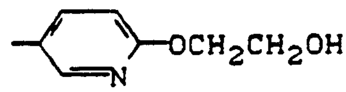 |
| 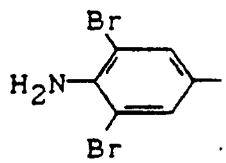 | 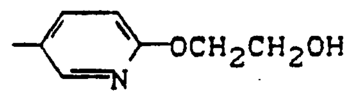 |
| 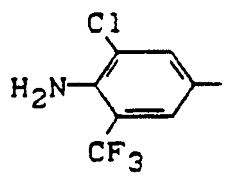 | 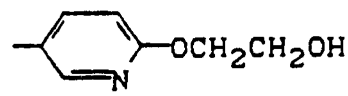 |
| 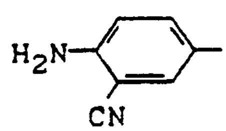 | 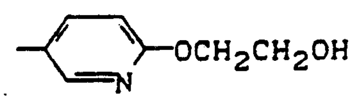 |
| 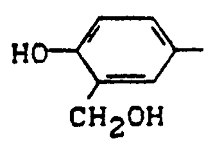 | 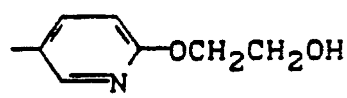 |
| 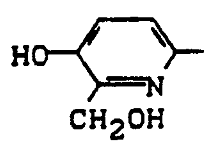 | 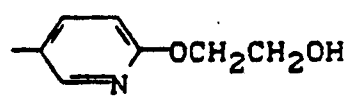 |

| 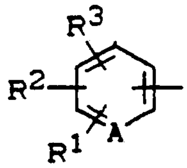 | 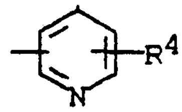 |
|---|---|
| 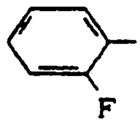 | 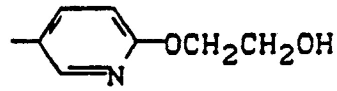 |
| 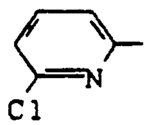 | 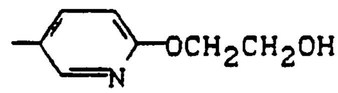 |
| 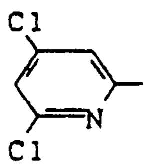 | 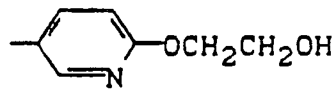 |
| 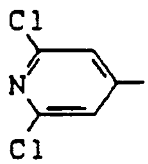 | 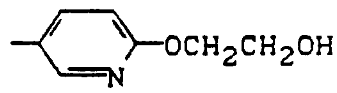 |

| 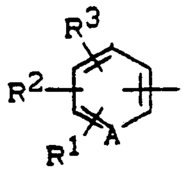 | 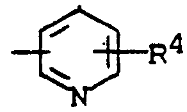 |
| --- | --- |
| 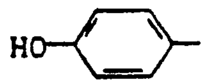 | 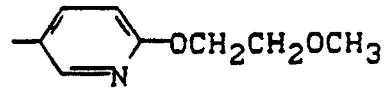 |
| 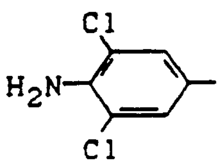 | 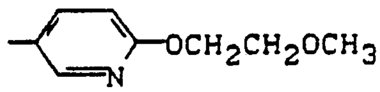 |
| 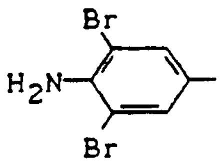 | 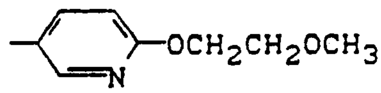 |
| 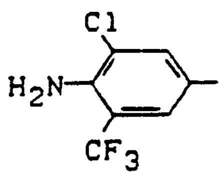 | 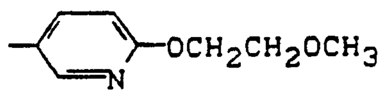 |
| 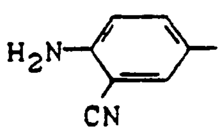 | 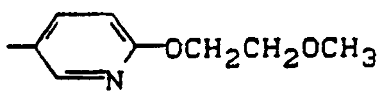 |
| 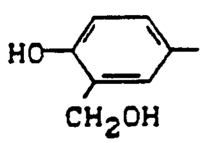 | 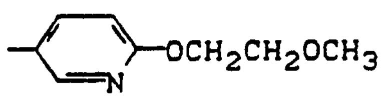 |
| 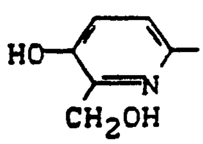 | 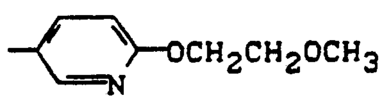 |

| 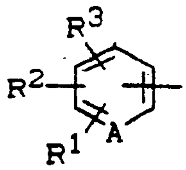 | 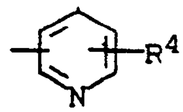 |
|---|---|
| 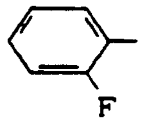 | 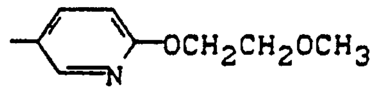 |
| 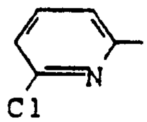 | 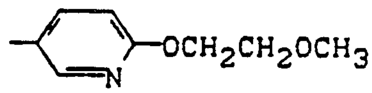 |
| 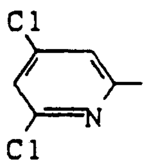 | 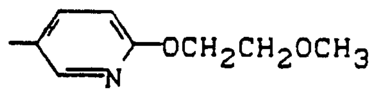 |
| 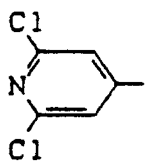 | 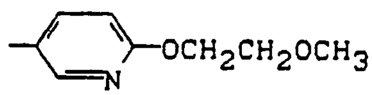 |

| 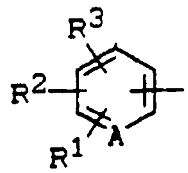 | 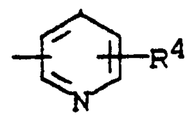 |
| --- | --- |
| 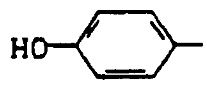 | 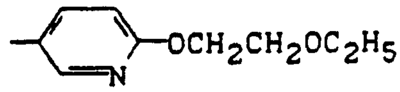 |
| 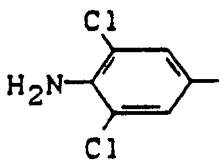 | 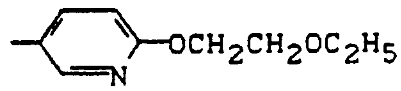 |
| 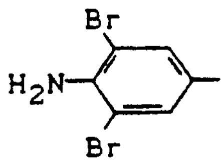 | 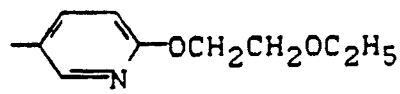 |
| 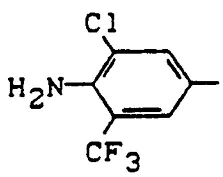 | 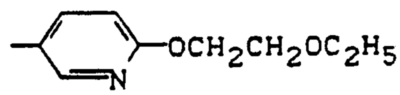 |
| 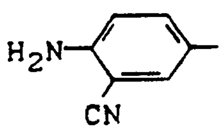 | 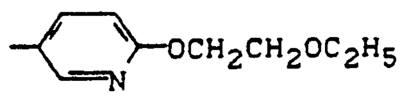 |
| 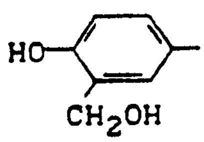 | 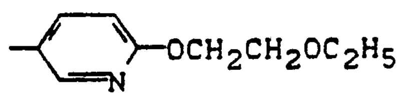 |
| 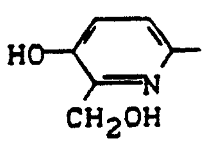 | 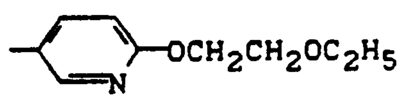 |

| 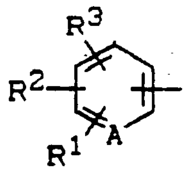 | 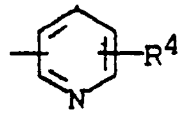 |
|---|---|
| 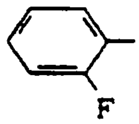 | 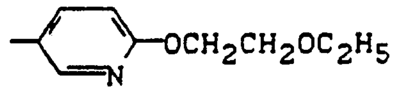 |
| 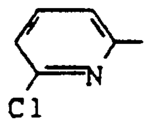 | 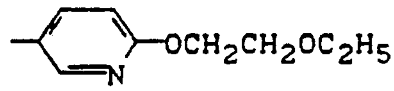 |
| 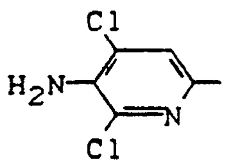 | 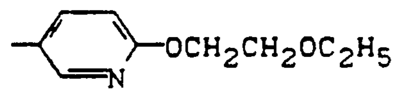 |
| 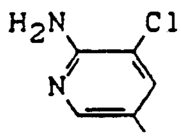 | 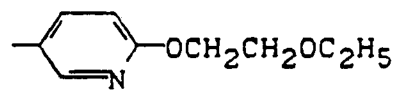 |

| 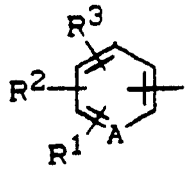 | 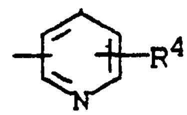 |
|---|---|
| 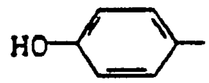 | 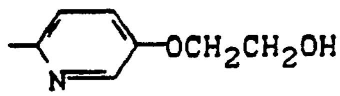 |
| 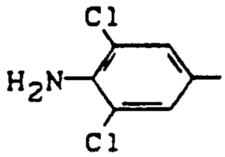 | 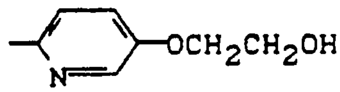 |
| 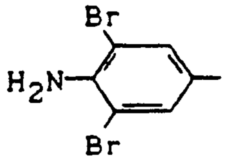 | 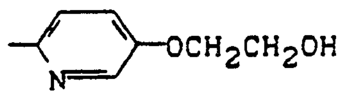 |
| 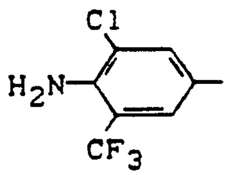 | 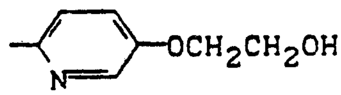 |
| 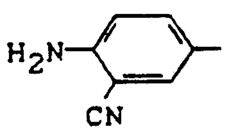 | 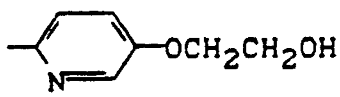 |
| 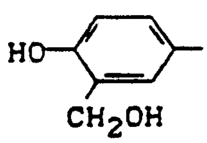 | 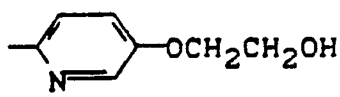 |
| 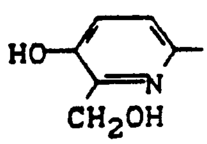 | 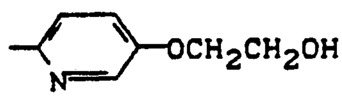 |

| 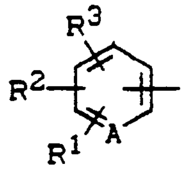 | 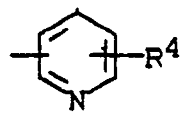 |
|---|---|
| 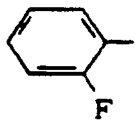 | 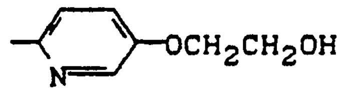 |
| 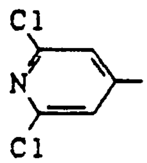 | 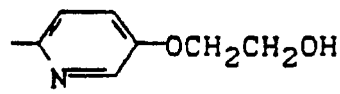 |
| 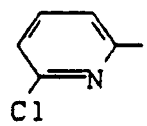 | 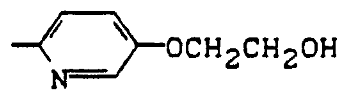 |
| 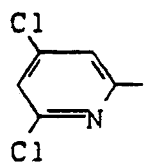 | 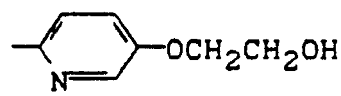 |

| 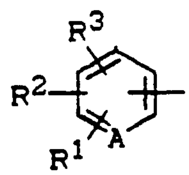 | 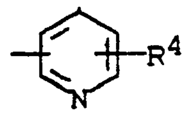 |
|---|---|
| 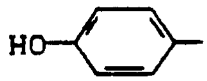 | 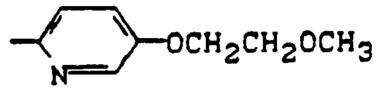 |
| 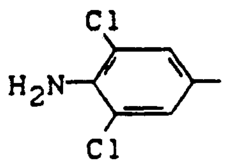 | 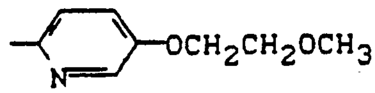 |
| 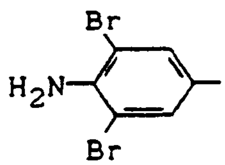 | 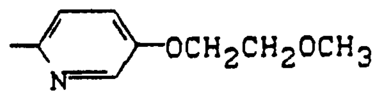 |
| 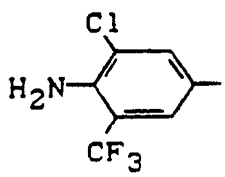 | 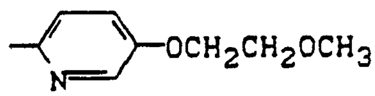 |
| 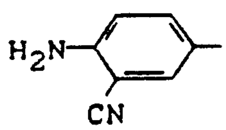 | 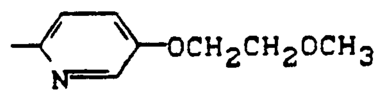 |
| 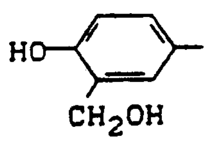 | 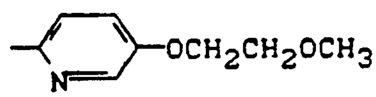 |
| 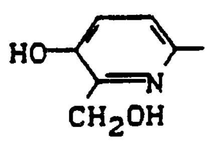 | 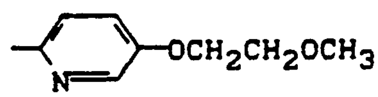 |

| 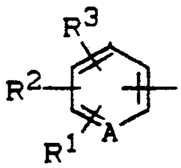 | 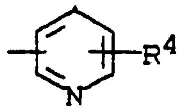 |
| --- | --- |
| 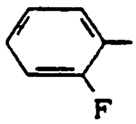 | 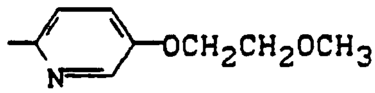 |
| 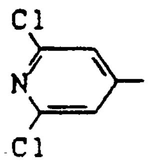 | 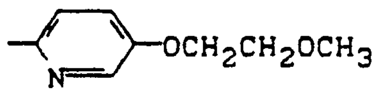 |
| 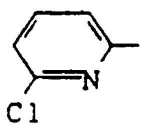 | 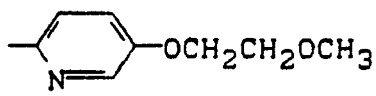 |
| 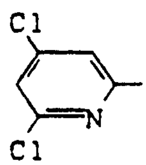 | 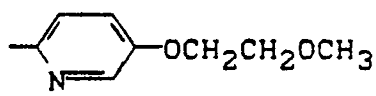 |

| 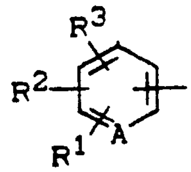 | 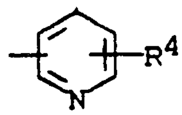 |
|---|---|
| 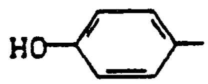 | 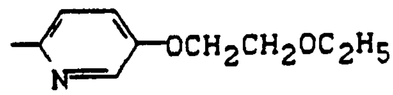 |
| 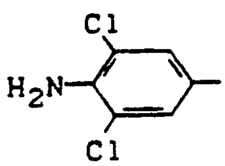 | 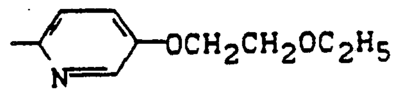 |
| 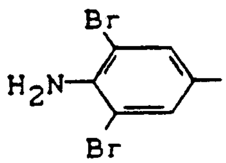 | 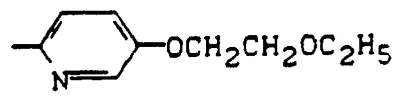 |
| 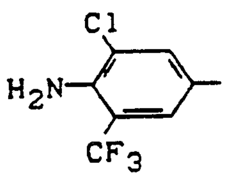 | 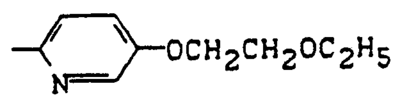 |
| 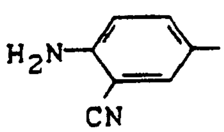 | 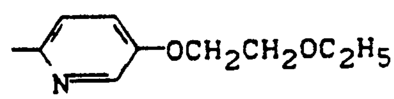 |
| 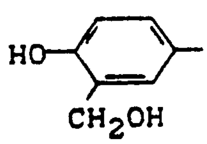 | 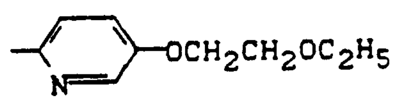 |
| 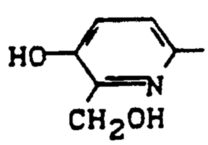 | 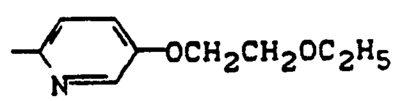 |

| 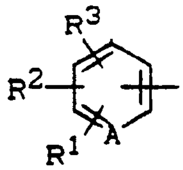 | 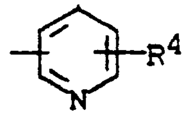 |
| --- | --- |
| 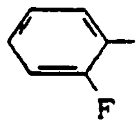 | 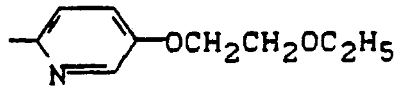 |
| 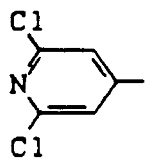 | 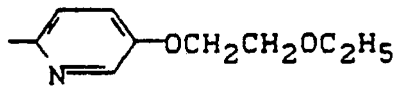 |
| 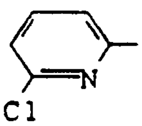 | 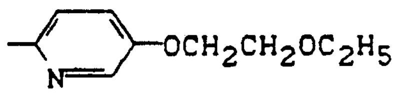 |
| 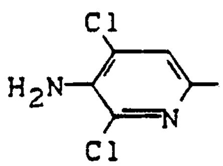 | 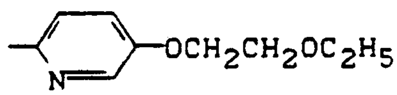 |

| 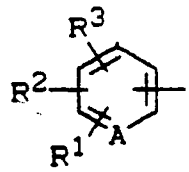 | 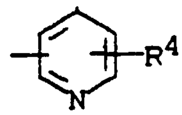 |
|---|---|
| 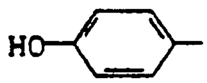 | 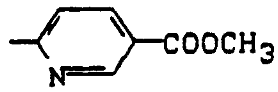 |
| 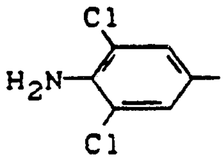 | 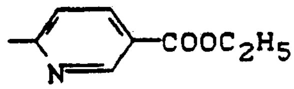 |
| 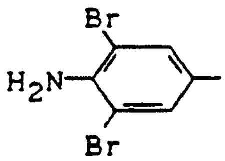 | 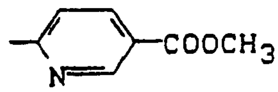 |
| 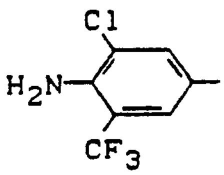 | 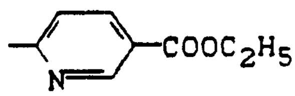 |
| 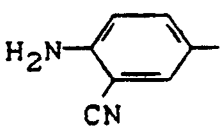 | 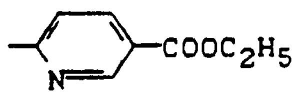 |
| 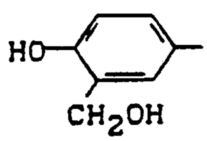 | 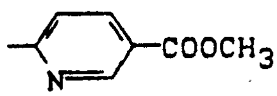 |
| 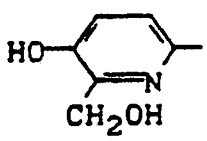 | 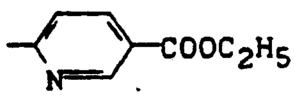 |

| 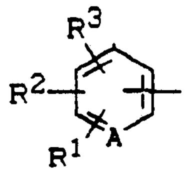 | 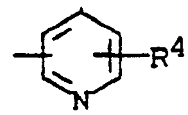 |
|---|---|
| 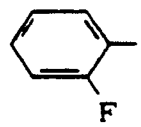 | 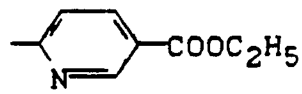 |
| 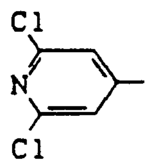 | 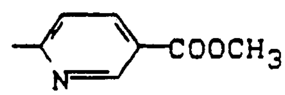 |
| 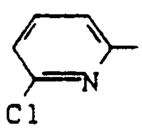 | 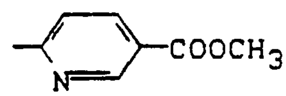 |
| 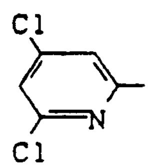 | 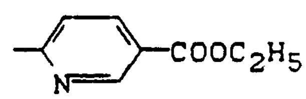 |

Weiterhin seien neben den Beispielen die folgenden Verbindungen der Formel I genannt:

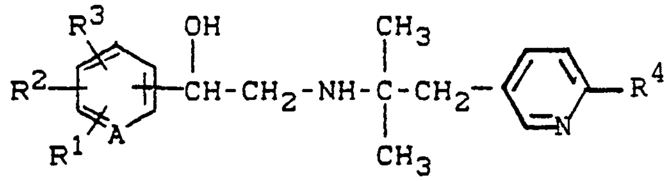

(I)

| 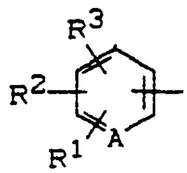 | $R^4$ |
| --- | --- |
| 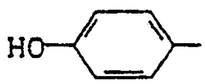 | $-OCH_2CH_2OCH_3$ |
| 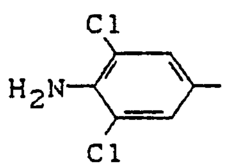 | $-OCH_2CH_2OCH_3$ |
| 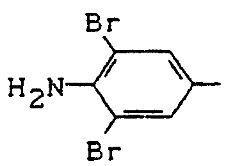 | $-OCH_2CH_2OCH_3$ |
| 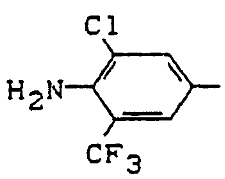 | $-OCH_2CH_2OCH_3$ |
| 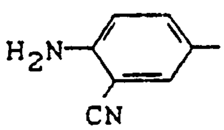 | $-OCH_2CH_2OCH_3$ |
| 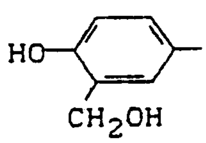 | $-OCH_2CH_2OCH_3$ |

| 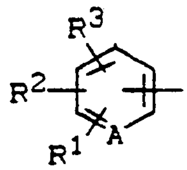 | $R^4$ |
|---|---|
| 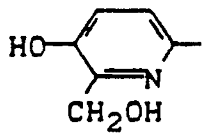 | $-OCH_2CH_2OCH_3$ |
| 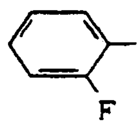 | $-OCH_2CH_2OCH_3$ |
| 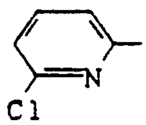 | $-OCH_2CH_2OCH_3$ |
| 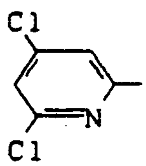 | $-OCH_2CH_2OCH_3$ |
| 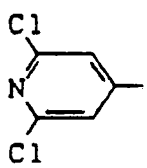 | $-OCH_2CH_2OCH_3$ |

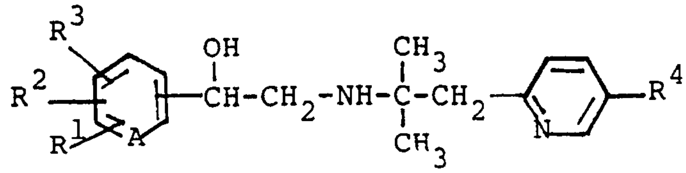

| 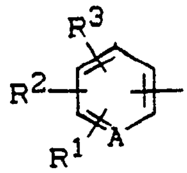 | $R^4$ |
| --- | --- |
| 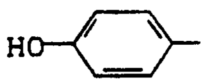 | $-OCH_2CH_2OC_2H_5$ |
| 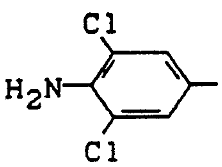 | $-OCH_2CH_2OC_2H_5$ |
| 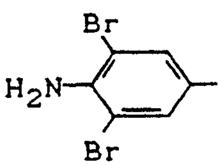 | $-OCH_2CH_2OC_2H_5$ |
| 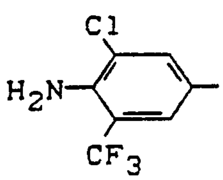 | $-OCH_2CH_2OC_2H_5$ |
| 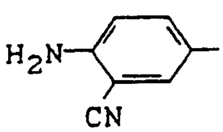 | $-OCH_2CH_2OC_2H_5$ |
| 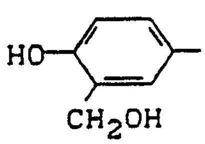 | $-OCH_2CH_2OC_2H_5$ |
| 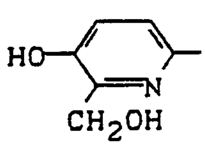 | $-OCH_2CH_2OC_2H_5$ |

| 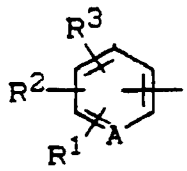 | $R^4$ |
|---|---|
| 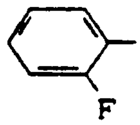 | $-OCH_2CH_2OC_2H_5$ |
| 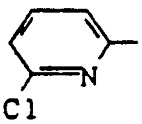 | $-OCH_2CH_2OC_2H_5$ |
| 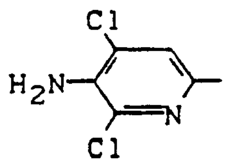 | $-OCH_2CH_2OC_2H_5$ |
| 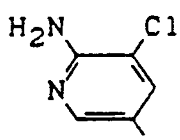 | $-OCH_2CH_2OC_2H_5$ |

Bevorzugt seien die Salze mit Salzsäure, Schwefelsäure, Oxalsäure, Maleinsäure, Fumarsäure, Malonsäure, Benzoesäure genannt.

Die Verbindungen der Formel (I) lassen sich nach den oben unter 2. angegebenen Verfahren a) bis f) herstellen.

Setzt man bei Verfahren 2a) als Halogenmethylketon der Formel (II) 2-Fluor-3-bromacetylbenzol und als Amin der Formel (III) 3-(5-Methoxycarbonyl-2-pyridyl)-2-propylamin ein, läßt sich Verfahren a) durch folgendes Reaktionsschema wiedergeben:

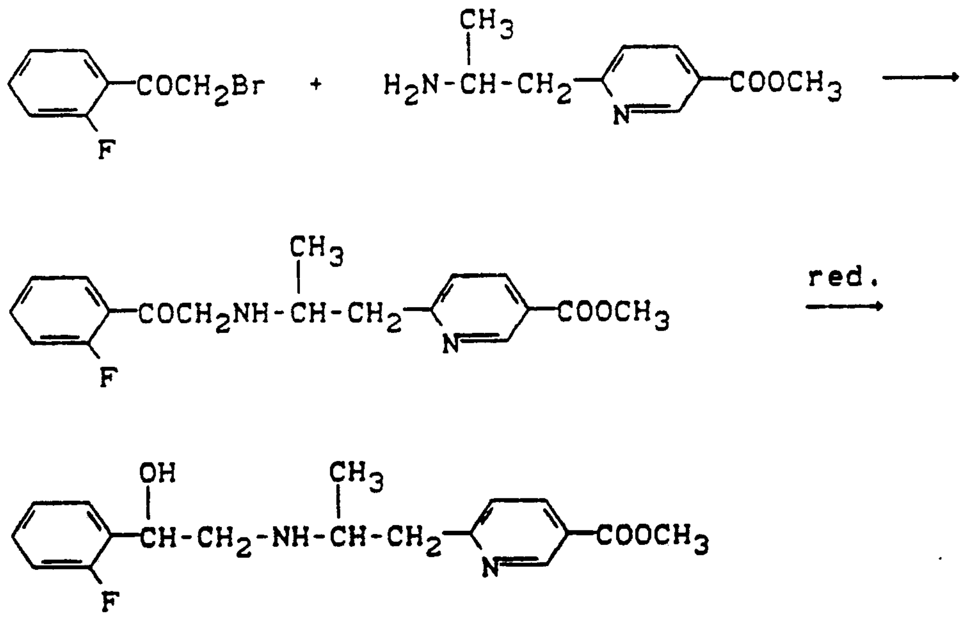


Verbindungen der Formel (II) sind bekannt (vgl. z.B. DE-OS 3 615 293, US-Pat. 4 522 822, EP-OS 23 385). Die Substituenten $R^1$, $R^2$, $R^3$ und A in Formel (II) besitzen bevorzugt die weiter oben angegebenen bevorzugten Bedeutungen. Im einzelnen seien folgende Verbindungen der Formel (II) genannt:
(2-Amino-3-chlor-5-pyridyl)-chlormethylketon,
(2-Amino-3-cyano-5-pyridyl)-chlormethylketon,
(2,4-Dichlor-3-amino-6-pyridyl)-brommethylketon,
(2-Cyano-3-amino-6-pyridyl)-brommethylketon,
(3-Amino-4-cyano-6-pyridyl)-brommethylketon,
(3-Amino-4-cyano-6-pyridyl)-chlormethylketon,
(2-Cyano-3-amino-4-chlor-6-pyridyl)-brommethylketon,
(2-Cyano-3-amino-4-chlor-6-pyridyl)-chlormethylketon,
(2-Chlor-3-amino-4-trifluormethyl-6-pyridyl)-brommethylketon,
(2-Trifluormethyl-3-amino-4-cyano-6-pyridyl)-brommethylketon,
(2-Fluor-3-amino-4-cyano-6-pyridyl)-chlormethylketon,
4-Amino-3,5-dichlorphenyl-brommethylketon,
4-Amino-3-chlor-5-trifluormethylphenyl-brommethylketon,
4-Amino-3-cyanophenyl-brommethylketon,
6-Chlor-2-pyridyl-brommethylketon,
4,6-Dichlor-2-pyridyl-brommethylketon,
2,6-Dichlor-4-pyridyl-brommethylketon,
3-Chlorphenyl-brommethylketon.

Amine der Formel (III) sind neu, wenn $R^4$ nicht für Wasserstoff steht. Ihre Herstellung wird weiter unten beschrieben. $R^0$ und $R^4$ besitzen bevorzugt die bei den Verbindungen der Formel (I) angegebenen Bedeutungen. Im einzelnen seien folgende Verbindungen der Formel (III) genannt:
1-[2-(2-Hydroxyethoxy)-5-pyridyl]-2-aminopropan,
1-[2-(2-Methoxyethoxy)-5-pyridyl]-2-aminopropan,
1-[2-(2-Ethoxyethoxy)-5-pyridyl]-2-aminopropan,
1-(2-Ethoxycarbonyl-5-pyridyl)-2-aminopropan,
1-[5-(2-Hydroxyethoxy)-2-pyridyl]-2-aminopropan,
1-[5-(2-Methoxyethoxy)-2-pyridyl]-2-aminopropan,
1-[5-(2-Ethoxyethoxy)-2-pyridyl]-2-aminopropan,
1-[2-(2-Methoxyethoxy)-5-pyridyl]-2-amino-2-methylpropan,
1-[2-(2-Ethoxyethoxy)-5-pyridyl]-2-amino-2-methylpropan.

Als Reduktionsmittel zur Durchführung des Verfahrens 2a) seien folgende Reduktionsmittel genannt:
$H_2$/Katalysator, als Katalysator seien beispielsweise genannt: $PtO_2$, Pd-Aktivkohle;
komplexe Metallhydride wie z.B. $LiAlH_4$, $NaBH_4$, $NaBH_3CN$.

Besonders bevorzugt werden folgende Reduktionsmittel eingesetzt:

$NaBH_4$ und $NaBH_3CN$

Verfahren 2a) wird durchgeführt, indem man Verbindungen (II) und (III) in einem Verdünnungsmittel in etwa äquimolaren Verhältnis zusammengibt.

Die Reaktion wird bei Temperaturen von -20 °C bis +100 °C bevorzugt durchgeführt.

Es wird bevorzugt bei Normaldruck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Petrolether, Ligroin, Benzol, Toluol; chlorierte Kohlenwasserstoffe wie Methylenchlorid, Ethylenchlorid, Chloroform; Ether wie Diethylether und Glykoldimethylether; Nitrile wie Acetonitril, Propionitril und Benzonitril; Alkohole wie Methanol, Ethanol, n- und i-Propanol.

Bevorzugt sind Alkohole, wie ohne Isolierung der Zwischenstufen sofort die Reduktion durchgeführt werden kann.

Setzt man bei Verfahren 2b) als Epoxid der Formel (IV) 3-Chlorphenylepoxid und als Amin der Formel (III) 1-[5-(2-Methoxyethoxy)-2-pyridyl]-2-aminopropan ein, läßt sich Verfahren 2b) durch folgendes Reaktionsschema wiedergeben:

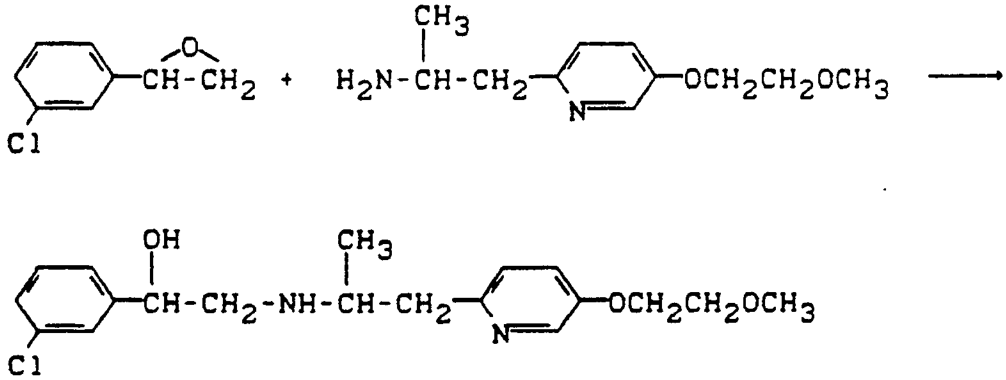

Epoxide der Formel (IV) sind bekannt (vgl. z.B. DE-OS 3 615 293; US-Pat. 4 522 822; EP 23 385). Die Substituenten $R^1$, $R^2$, $R^3$ und A besitzen bevorzugt die weiter oben angegebenen bevorzugten Bedeutungen. Im einzelnen seien die folgenden Epoxide genannt:

2-Amino-3-chlorpyridin-5-epoxid,
2-Amino-3-cyanopyridin-5-epoxid,
2,4-Dichlor-3-aminopyridin-6-epoxid,
2-Chlor-3-amino-4-cyanopyridin-6-epoxid,
2-Cyano-3-amino-4-chlorpyridin-6-epoxid,
2-Cyano-3-aminopyridin-6-epoxid,
2-Chlor-3-amino-4-trifluormethylpyridin-6-epoxid,
2-Brom-3-amino-4-cyanopyridin-6-epoxid,
4-Amino-3,5-dichlorphenyl-epoxid,
4-Amino-3-chlor-5-trifluormethylphenyl-epoxid,
4-Amino-3-cyanophenyl-epoxid,
2-Chlorpyridin-6-epoxid,
2,4-Dichlorpyridin-6-epoxid,
2,6-Dichlorpyridin-4-epoxid,

Verfahren 2b) wird durchgeführt, indem man etwa äquimolare Mengen des Epoxids der Formel IV und des Amins der Formel (III) in einem Verdünnungsmittel umsetzt.

Im allgemeinen wird ein Überschuß an Amin (1-3 molar), bevorzugt 1-1,5 molar), bezogen auf das Epoxid der Formel (IV), verwendet.

Die Reaktion wird bei Temperaturen von +20 °C bis +150 °C bevorzugt durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, ferner Nitrile wie Acetonitril und Benzonitril, Amide wie Dimethylformamid, Alkohole wie Methanol, Ethanol, n- und i-Propanol.

Bevorzugt sind Alkohole.

Setzt man bei Verfahren 2c) als β-Halogenmethylverbindung der Formel V 5-Methoxy-3-(1-hydroxy-2-chlorethyl)-pyridin und als Amin der Formel (III) 1-(5-Methoxy-2-pyridyl)-2-aminopropan ein, läßt sich Verfahren c) durch folgendes Reaktionsschema wiedergeben:

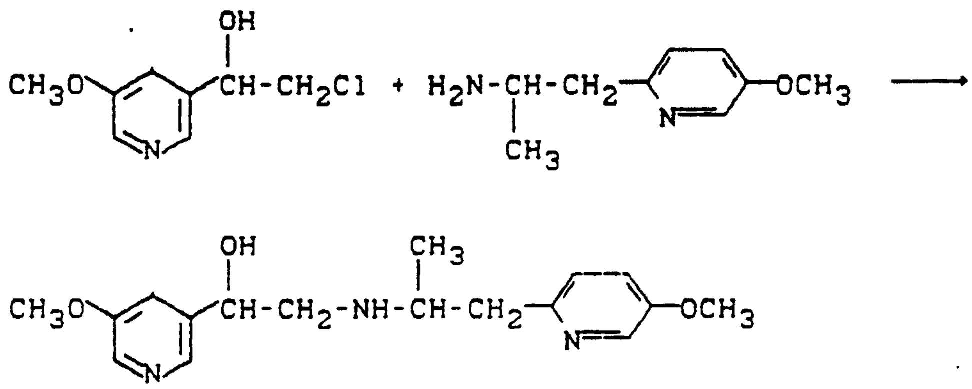

β-Halogenethylverbindungen der Formel (V) sind bekannt (vgl. z.B. DE-OS 3 615 293, US-Pat. 4 522 822, EP 23 385). Die Substituenten $R^1$, $R^2$, $R^3$ und A in Formel (II) besitzen bevorzugt die bei den Verbindungen der Formel (I) angegebenen bevorzugten Bedeutungen.

Im einzelnen seien folgende Verbindungen der Formel (V) genannt:

1-(2-Amino-3-chlor-5-pyridyl)-2-chlorethanol,
1-(2-Amino-3-cyano-5-pyridyl)-chlorethanol,
1-(2,4-Dichlor-3-amino-6-pyridyl)-2-chlorethanol,
1-(2-Chlor-3-amino-4-cyano-6-pyridyl)-chlorethanol,
1-(2-Cyano-3-amino-4-chlor-6-pyridyl)-brommethanol,
1-(2-Cyano-3-amino-6-pyridyl)-2-chlorethanol,
1-(3-Amino-4-cyano-6-pyridyl)-2-bromethanol,
1-(2-Chlor-3-amino-4-trifluormethyl-6-pyridyl)-2-chlorethanol,
1-(4-Amino-3,5-dichlorphenyl)-2-bromethanol,
1-(4-Amino-3-chlor-5-trifluormethylphenyl)-2-chlorethanol,
1-(4-Amino-3-cyanophenyl)-2-bromethanol,
1-(6-Chlor-2-pyridyl)-2-bromethanol,
1-(4,6-Dichlor-2-pyridyl)-2-bromethanol,
1-(2,6-Dichlor-4-pyridyl)-2-bromethanol,
1-(3-Chlorphenyl)-2-bromethanol,
1-(2-Fluorphenyl)-2-bromethanol.

Verfahren 2c) wird durchgeführt, indem man die beta-Halogenethylverbindung der Formel V mit überschüssigem Amin der Formel (III), gegebenenfalls in Gegenwart eines Verdünnungsmittels, umsetzt.

Die Reaktion wird bei Temperaturen von +20 bis +150° C durchgeführt.

Es wird bei Normaldruck oder unter erhöhtem Druck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe wie Pentan, Hexan, Cyclohexan, Benzol, Toluol, Methylenchlorid, Chloroform, ferner Ether wie Diethylether, Tetrahydrofuran und Dioxan, weiterhin Nitrile wie Acetonitril und Benzonitril, ferner Amide wie Dimethylformamid, ferner Alkohole wie Methanol, Ethanol, n- und i-Propanol.

Bevorzugt eingesetzt werden Alkohole.

Setzt man bei Verfahren 2d) als Verbindung der Formel VI 1-(5-Fluor-3-pyridyl)-2-aminoethanol und als Verbindung der Formel (VII) (5-Methoxy-2-pyridyl)-aceton ein, läßt sich das Verfahren durch folgendes Formelschema wiedergeben:

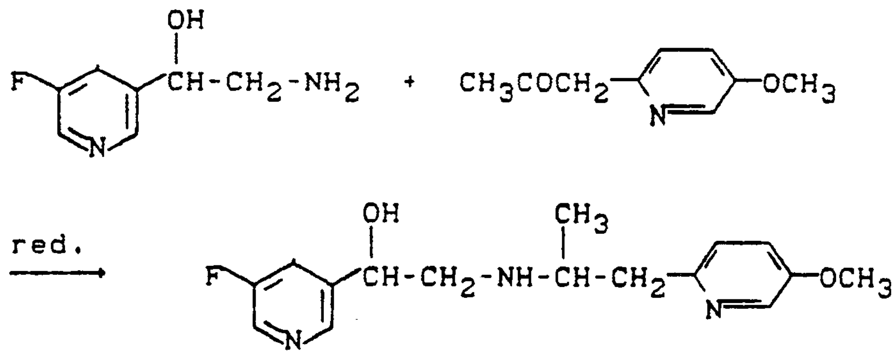

Verbindungen der Formel (VI) sind bekannt (vgl. z.B. DE-OS 3 615 293, US-Pat. 4 522 822, EP 23 385, DE-OS 3 627 663). Die Substituenten $R^1$, $R^2$, $R^3$ und A in Formel (II) besitzen bevorzugt die bei den Verbindung der Formel (I) angegebenen bevorzugten Bedeutungen.

Im einzelnen seien folgende Verbindungen der Formel (VI) genannt:
1-(2-Amino-3-chlor-5-pyridyl)-2-aminoethanol,
1-(2-Amino-3-cyano-5-pyridyl)-2-aminoethanol,
1-(2,4-Dichlor-3-amino-6-pyridyl)-2-aminoethanol,
1-(2-Chlor-3-amino-4-cyano-6-pyridyl)-2-aminoethanol,
1-(2-Cyano-3-amino-6-pyridyl)-aminoethanol,
1-(2-Chlor-3-amino-4-trifluormethyl-6-pyridyl)-2-aminoethanol,
1-(4-Amino-3,5-dichlorphenyl)-2-aminoethanol,
1-(4-Amino-3-chlor-5-trifluormethylphenyl)-2-aminoethanol,
1-(4-Amino-3-cyanophenyl)-2-aminoethanol,
1-(6-Chlor-2-pyridyl)-2-aminoethanol,
1-(4,6-Dichlor-2-pyridyl)-2-aminoethanol,
1-(2,6-Dichlor-4-pyridyl)-2-aminoethanol,
1-(3-Chlorphenyl)-2-aminoethanol,
1-(2-Fluorphenyl)-2-aminoethanol.

Verbindungen der Formel (VII) sind teilweise neu. Die Herstellung der neuen Verbindungen wird weiter unten beschrieben. Der Rest $R^4$ besitzt bevorzugt die weiter oben angegebenen bevorzugten Bedeutungen.

Im einzelnen seien folgende Verbindungen der Formel (VII) genannt.
[5-(2-Hydroxyethoxy)-2-pyridyl]-aceton,
[5-(2-Methoxyethoxy)-2-pyridyl]-aceton,
[5-(2-Ethoxyethoxy)-2-pyridyl]-aceton,
(5-Ethoxycarbonyl-2-pyridyl]-aceton,
[2-(2-Hydroxyethoxy)-5-pyridyl]-aceton,
[2-(2-Methoxyethoxy)-5-pyridyl]-aceton,
[2-(2-Ethoxyethoxy)-5-pyridyl]-aceton.

Verfahren 2d) wird durchgeführt, indem man etwa äquimolare Mengen der Verbindungen der Formeln (VI) und (VII) in einem Verdünnungsmittel vorlegt und die Mischung reduziert.

Die Reaktion wird bei Temperaturen von 0 °C bis 150 °C durchgeführt.

Es wird bevorzugt bei Normaldruck gearbeitet.

Als Verdünnungsmittel dienen alle inerten organischen Lösungsmittel. Hierzu gehören aliphatische und aromatische, gegebenenfalls halogenierte Kohlenwasserstoffe wie Pentan, Hexan, Cyclohexan, Benzol, Toluol, Methylenchlorid, Chloroform, Chlorbenzol, ferner Ether wie Diethylether, Tetrahydrofuran, Dioxan, weiterhin Nitrile wie Acetonitril und Benzonitril, ferner Amide wie Dimethylformamid, ferner Alkohole wie Methanol, Ethanol.

Als Reduktionsmittel dienen:
$H_2$/Katalysator, als Katalysator sei beispielsweise $PtO_2$ genannt; komplexe Metallhydride wie z.B. $LiAlH_4$, $NaBH_3CN$.

Setzt man bei Verfahren 2e) als Verbindung der Formel (VIII) 2-Fluorphenylglyoxal und als Amin der Formel (III) 1-[5-(2-Methoxyethoxy)-2-pyridyl)-2-aminopropan ein, läßt sich das Verfahren durch folgendes Reaktionsschema wiedergeben:

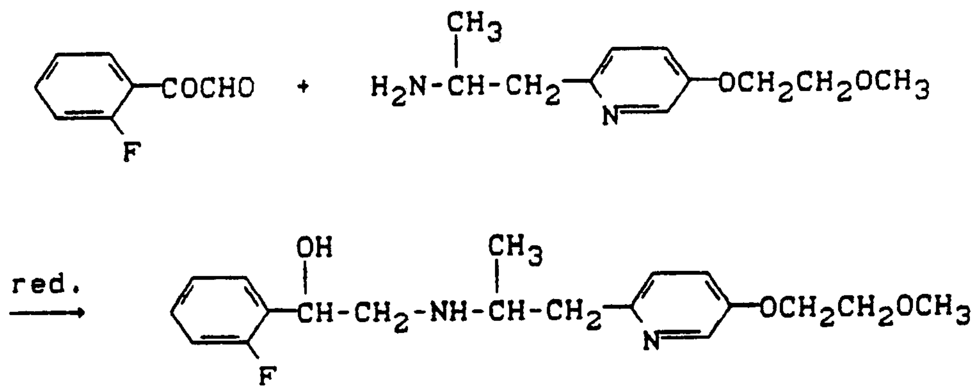


Verbindungen der Formel (VIII) sind bekannt (vgl. z.B. DE-OS 3 615 293, US-Pat. 4 522 822, EP 23 385). Die Substituenten $R^1$, $R^2$, $R^3$ und A in Formel (VIII) besitzen bevorzugt die weiter oben bei den Verbindungen der Formel (I) angegebenen bevorzugten Bedeutungen. Im einzelnen seien folgende Verbindungen der Formel (VIII) genannt:

2-Amino-3-chlor-5-pyridylglyoxal,
2-Amino-3-cyano-5-pyridylglyoxal,
2,4-Dichlor-3-amino-6-pyridylglyoxal,
2-Cyano-3-amino-6-pyridylglyoxal,
2-Chlor-3-amino-4-trifluormethyl-6-pyridylglyoxal,
4-Amino-3,5-dichlor-phenylglyoxal,
4-Amino-3-Chlor-5-trifluormethyl-phenylglyoxal,
4-Amino-3-cyano-phenylglyoxal,
6-Chlor-2-pyridylglyoxal,
4,6-Dichlor-2-pyridylglyoxal,
2,6-Dichlor-4-pyridylglyoxal,
3-Chlor-phenylglyoxal,
2-Fluor-phenylglyoxal.

Verfahren 2e) wird durchgeführt, indem man zur Verbindung der Formel (VIII) in einem Verdünnungsmittel etwa die äquivalente Menge des Amins der Formel (III) zusetzt und anschließend reduziert.

Die Reaktion wird bei Temperaturen von 0° C bis 100° C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, außerdem Ester wie Essigsäuremethylester und -ethylester, ferner Nitrile wie z.B. Acetonitril und Propionitril, Benzonitril, darüber hinaus Amide wie z.B. Dimethylformamid, Dimethylacetamid und N-Methylpyrrolidon sowie Tetramethylensulfon und Hexamethylphosphorsäuretriamid, weiterhin Alkohole wie Methanol, Ethanol, n- und i-Propanol.

Als Reduktionsmittel dienen $H_2$/Katalysator; als Katalysator seien $PtO_2$ und Pd-Kohle genannt, ferner komplexe Metallhydride wie $LiAlH_4$ und $NaBH_4$.

Setzt man bei Verfahren 2f) als Verbindung der Formel (IX) 3-Chlormandelsäure-2-[3-(5-methoxy-2-pyridyl)]-propylamid ein, läßt sich das Verfahren durch folgendes Formelschema wiedergeben:

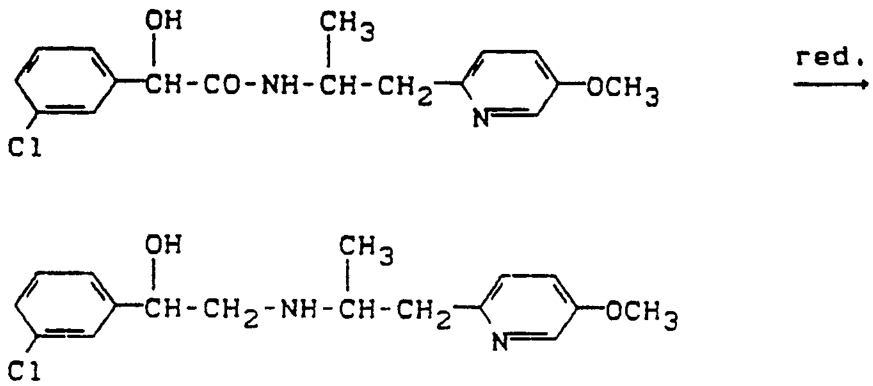


Verbindungen der Formel (IX) sind neu. Ihre Herstellung wird weiter unten beschrieben. Die Substituenten $R^0$ bis $R^4$ und A besitzen bevorzugt die weiter oben bei den Verbindungen der Formel (I) angegebenen bevorzugten Bedeutungen. Im einzelnen seien folgende Verbindungen genannt:
3-Chlormandelsäure-2-[3-[5-(2-methoxyethoxy)]-2-pyridyl-propylamid,
2-Fluormandelsäure-2-[3-[5-(2-methoxyethoxy)]-2-pyridyl]propylamid,
(6-Chlor-2-pyridyl)hydroxyessigsäure-2-[3-[5-(2-methoxyethoxy)]-2-pyridyl]propylamid,
(4,6-Dichlor-2-pyridyl)hydroxyessigsäure-2-[3-[5-(2-ethoxyethoxy)]-2-pyridyl]propylamid,
(2,6-Dichlor-4-pyridyl)hydroxyessigsäure-2-[3-[5-(2-methoxyethoxy)j]-2-pyridyl]propylamid,
3-Chlormandelsäure-2-[3-[-(2-methoxyethoxy)]-5-pyridyl]-propylamid,
(6-Chlor-2-pyridyl)hydroxyessigsäure-2-[3-[2-(2-ethoxyethoxy)]-5-pyridyl]-propylamid.

Verfahren 2f) wird durchgeführt, indem man die Verbindung der Formel (IX) in einem Verdünnungsmittel mit überschüssigem Reduktionsmittel umsetzt.

Die Reaktion wird bei Temperaturen von 0° C bis +150° C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan.

Als Reduktionsmittel dienen komplexe Metallhydride wie $LiAlH_4$, Borane wie Diboran.

Setzt man bei Verfahren 4 a als Verbindung der Formel (VII) (2-Methoxy-5-pyridyl)aceton ein, läßt sich das Verfahren durch folgendes Reaktionsschema wiedergeben:

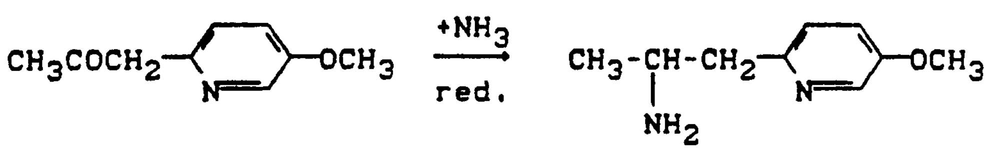


Verbindungen der Formel (VII) sind teilweise neu. Die Herstellung der neuen Verbindungen wird weiter unten beschrieben. Der Substituent $R^4$ besitzt bevorzugt die weiter oben bei den Verbindungen der Formel (I) genannten bevorzugten Bedeutungen und steht in p-Position zur Acetonylgruppe. Im einzelnen seien folgende Verbindungen der Formel (VII) genannt:
[5-(2-Hydroxyethoxy)-2-pyridyl]-aceton,
[5-(2-Methoxyethoxy)-2-pyridyl]-aceton,
[5-(2-Ethoxyethoxy)-2-pyridyl]-aceton,
(5-Ethoxycarbonyl-2-pyridyl)-aceton,
[2-(2-Hydroxyethoxy)-5-pyridyl]-aceton,
[2-(2-methoxyethoxy)-5-pyridyl]-aceton,
[2-(2-Ethoxyethoxy)-5-pyridyl]-aceton,

Das Verfahren wird durchgeführt, indem man in an sich bekannter Weise

a) eine Verbindung der Formel (VII) in Gegenwart von überschüssigem Ammoniak katalytisch reduziert (vgl. Houben-Weyl, Methoden der org. Chemie, Bd. 11/1, S. 602 ff),

b) eine Verbindung der Formel (VII) in ihr Oxim, ihren Oximether oder -ester überführt und diese

beispielsweise mit $NaBH_4$, $NaBH_3CN$, $LiAlH_4$ oder Diboran reduziert (vgl. J. Org. Chem. 34 (1969), 1817).

Setzt man bei Verfahren 4 b als Verbindung der Formel XXII 1-(2-Methoxy-5-pyridyl)-2-formylamino-2-methylpropan ein, läßt sich das Verfahren durch folgendes Formelschema wiedergeben.

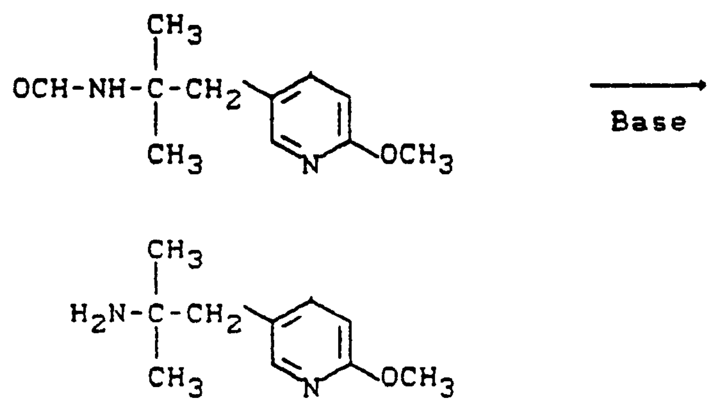


Die Verbindungen der Formel XXII sind neu. Ihre Herstellung wird weiter unten bei Verfahren 24 beschrieben. Bevorzugt werden Verbindungen der Formel XXII eingesetzt, in denen $R^4$ die weiter oben bei den Verbindungen der Formel I angegebenen bevorzugten und besonders bevorzugten Bedeutungen besitzt und $R^{11}$ für die Reste -NHCHO und $-NHCOCH_3$ steht. Im einzelnen seien folgende Verbindungen der Formel XXII genannt:

1-[2-(2-Methoxyethoxy)-5-pyridyl]-2-formylamino-2-methylpropan
1-[2-(2-Ethoxyethoxy)-5-pyridyl]-2-formylamino-2-methylpropan
1-[2-(2-Ethoxyethoxy)-5-pyridyl]-2-acetamino-2-methylpropan

Verfahren 4 b wird durchgeführt, indem man Verbindungen der Formel XXII bevorzugt in Gegenwart wasserlöslicher organischer Verdünnungsmittel und anorganischen Basen umsetzt.

Als Basen seien Alkali- und Erdalkalihydroxide, -carbonate, -hydrogencarbonate genannt.

Die Basen werden äquimolar oder in bis zu 20-fachem, bevorzugt 3- bis 10-fachem Überschuß, bezogen auf die Verbindungen der Formel XXII, eingesetzt.

Als Verdünnungsmittel dienen bevorzugt wassermischbare organische Lösungsmittel wie Alkohole, z.B. Methanol, Ethanol, Dioxan, Ethylenglycoldimethylether, Diethylenglycoldimethylether, Dimethylformamid, Dimethylsulfoxid, Sulfolan, N-Methylpyrrolidon. Es wird bei Temperaturen von 20 bis 200 °C, bevorzugt 50 bis 180 °C, gearbeitet.

Setzt man bei Verfahren 6) als Verbindung der Formel (X) (2-Fluorphenyl)acetoxyessigsäure-2-[3-[5-(2-methoxyethoxy)-2-pyridyl]]-propylamid ein, läßt sich das Verfahren durch folgendes Reaktionsschema wiedergeben:

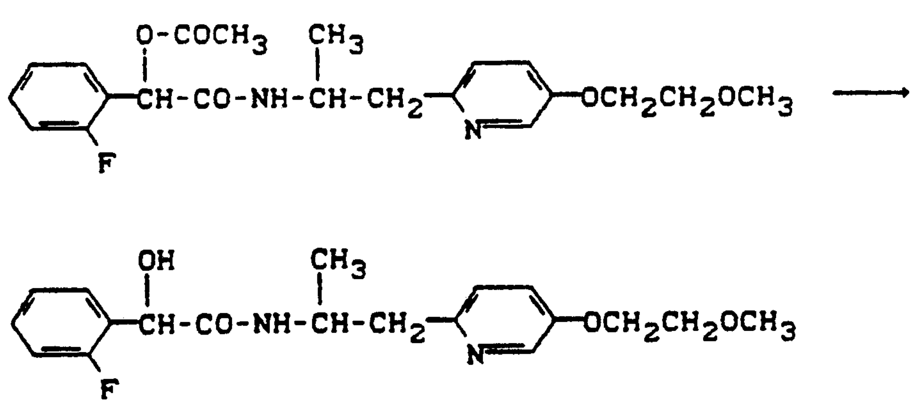

Verbindungen der Formel (X) sind neu. Ihre Herstellung wird weiter unten beschrieben. Die Substituenten $R^0$ bis $R^4$ und A besitzen bevorzugt die weiter oben bei den Verbindungen der Formel (I) angegebenen bevorzugten Bedeutungen. Im einzelnen seien folgende Verbindungen der Formel (X) genannt:

(3-Chlorphenyl)acetoxyessigsäure-2-[3-[5-(2-methoxyethoxy)]-2-pyridyl-propylamid,
(2-Fluorphenyl)acetoxyessigsäure-2-[3-[5-(2-ethoxyethoxy)]-2-pyridyl]-propylamid,

(6-Chlor-2-pyridyl)acetoxyessigsäure-2-[3-[5-(2-methoxyethoxy)]-2-pyridyl]-propylamid,
(4,6-Dichlor-2-pyridyl)acetoxyessigsäure-2-[3-[5-(2-ethoxyethoxy)]-2-pyridyl]-propylamid,
(2,6-Dichlor-4-pyridyl)acetoxyessigsäure-2-[3-[5-(2-methoxyethoxy)]-2-pyridyl]-propylamid,
(3-Chlorphenyl)acetoxyessigsäure-2-[3-[2-methoxyethoxy)]-5-pyridyl]-propylamid,
(6-Chlor-2-pyridyl)acetoxyessigsäure-2-[3-[2-(2-ethoxyethoxy)]-5-pyridyl]-propylamid.

Zur Abspaltung der Acetylgruppe werden anorganische Säuren oder Basen verwendet. Hierzu zählen Halogenwasserstoffsäuren wie Salzsäure, Schwefelsäure, Phosphorsäure, oder Basen wie NaOH, KOH.

Das Verfahren wird durchgeführt, indem man die Verbindung (X) in einem Verdünnungsmittel als Lösungsvermittler mit überschüssiger wäßriger Lösung der anorganischen Säure oder Base behandelt.

Die Reaktion wird bei Temperaturen von +20° C bis +150° C durchgeführt.

Es wird bevorzugt bei Normaldruck gearbeitet.

Als Verdünnungsmittel können alle inerten organischen Lösungsmittel, die mit Wasser mischbar sind, verwendet werden. Hierzu zählen Ether wie Tetrahydrofuran, Dioxan, Nitrile wie Acetonitril, Amide wie Dimethylformamid, Alkohole wie Methanol, Ethanol, Dimethylsulfoxid.

Setzt man bei Verfahren 8) als Verbindung der Formel (XI) 2-Fluorbenzaldehyd und als Isonitril der Formel (XII) 3-(5-Methoxy-2-pyridyl)-2-propylisonitril ein, läßt sich Verfahren 8) durch folgendes Formelschema wiedergeben:

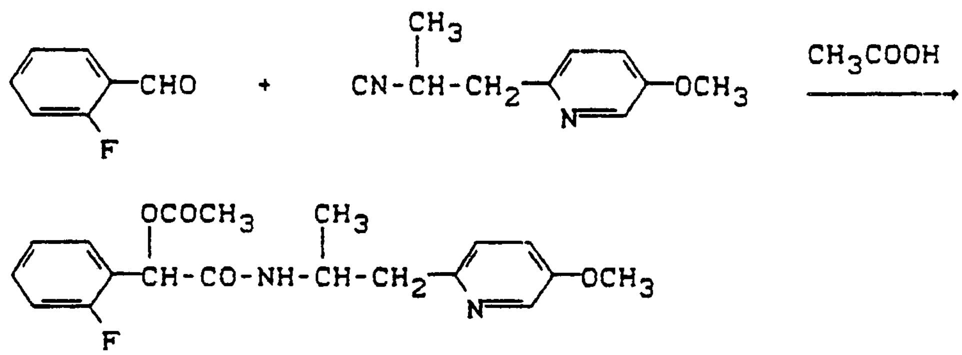


Verbindungen der Formel (XI) sind bekannt (vgl. z.B. DE-OS 3 615 293, US-Pat. 4 522 822, EP 23 385). Die Substituenten $R^1$ bis $R^3$ und A besitzen bevorzugt die weiter oben bei den Verbindungen der Formel (I) angegebenen bevorzugten Bedeutungen. Im einzelnen seien folgende Verbindungen der Formel (XI) genannt:
2-Chlorpyridin-4-aldehyd,
2-Cyanopyridin-4-aldehyd,
2, 4-Dichlorpyridin-4-aldehyd,
2-Aminopyridin-5-aldehyd,
2-Amino-3-chlorpyridin-5-aldehyd,
2-Amino-3-cyanopyridin-5-aldehyd,
2-Chlor-3-aminopyridin-6-aldehyd,
2-Cyano-3-aminopyridin-6-aldehyd,
2,4-Dichlor-3-aminopyridin-6-aldehyd,
3-Amino-4-cyanopyridin-6-aldehyd,
2-Chlorpyridin-6-aldehyd,
2,4-Dichlorpyridin-6-aldehyd,
3-Chlorbenzaldehyd,
4-Amino-3,5-dichlorbenzaldehyd,
4-Amino-3-cyanobenzaldehyd.

Isonitrile der Formel (XII) sind neu. Ihre Herstellung wird weiter unten (Verfahren 10) beschrieben. Die Substituenten $R^0$ und $R^4$ besitzen bevorzugt die weiter oben bei den Verbindungen der Formel (I) angegebenen bevorzugten Bedeutungen. Im einzelnen seien folgende Verbindungen der Formel (XII) genannt:
3-[2-(2-Methoxyethoxy)-5-pyridyl]-2-propylisonitril,
3-[2-(2-Ethoxyethoxy)-5-pyridyl]-2-propylisonitril,
3-[5-(2-Methoxyethoxy)-2-pyridyl]-2-propylisonitril,
3-[5-(2-Ethoxyethoxy)-2-pyridyl]-2-propylisonitril.

Das Verfahren wird durchgeführt, indem man die Verbindung (XI) mit der doppelt molaren Menge des Isonitrils der Formel (XII) und Essigsäure in einem Verdünnungsmittel umsetzt.

Die Reaktion wird bei Temperaturen von 0° C bis +150° C durchgeführt.

Es wird bevorzugt bei Normaldruck gearbeitet.

Als Verdünnungsmittel dienen alle inerten organischen Lösungsmittel. Hierzu gehören insbesondere gegebenenfalls halogenierte aliphatische und aromatische Kohlenwasserstoffe wie Pentan, Hexan, Cyclohexan, Benzol, Toluol, Methylenchlorid, Chloroform, Chlorbenzol, Ether wie Diethylether, Tetrahydrofuran, Nitrile wie Acetonitril und Benzonitril.

Setzt man bei Verfahren 10) als Verbindung der Formel (III) 1-[5-(2-Methoxyethoxy)-2-pyridyl]-2-aminopropan ein; läßt sich das Verfahren durch folgendes Formelschema wiedergeben:

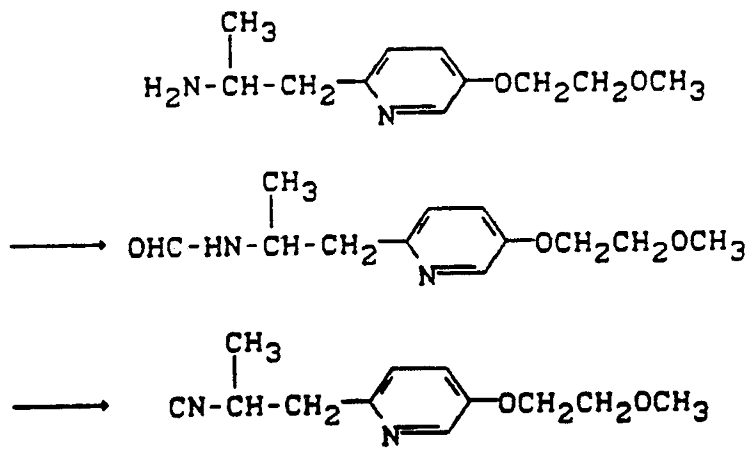

Die Herstellung der neuen Verbindungen der Formel III wurde bereits oben beschrieben.

Die Reaktionsführung erfolgt in an sich bekannter Weise, indem man beispielsweise ein Amin der Formel III zunächst in die entsprechende N-Formylverbindung überführt und diese anschließend z.B. mit Phosgen zum Isonitril umsetzt (vgl. hierzu: J. Ugi et. al., Angew. Chem. 77 (1965), 492).

Setzt man bei Verfahren 12a) als Verbindung der Formel (XIV) 5-(2-Methoxyethoxy)-2-methylpyridin ein, läßt sich das Verfahren durch folgendes Reaktionsschema wiedergeben:

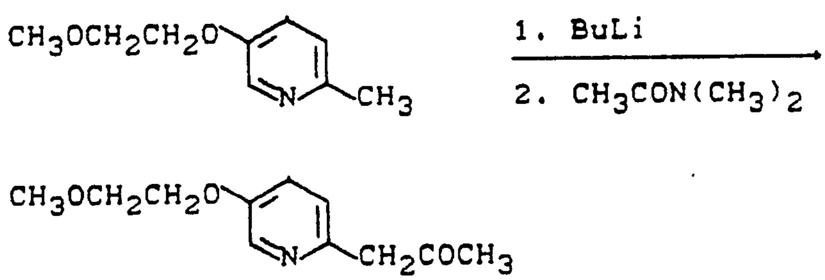


Verbindungen der Formel (XIV) sind teilweise neu. Die Herstellung der neuen Verbindungen sind weiter unten beschrieben. Die Substituenten $R^{10}$ und Z besitzen bevorzugt die weiter oben angegebenen bevorzugten Bedeutungen. Im einzelnen seien folgende Verbindungen der Formel (XIV) genannt:

5-Methoxy-2-methylpyridin,
5-Ethoxy-2-methylpyridin,
5-(2-Ethoxyethoxy)-2-methylpyridin.

Verfahren 12a) wird durchgeführt, indem man eine Verbindung der Formel (XIV) in einem Verdünnungsmittel mittels einer lithiumorganischen Verbindung lithiiert und anschließend mit N,N-Dimethylacetamid umsetzt. Als Verdünnungsmittel werden verwendet: gesättigte und ungesättigte aliphatische und aromatische Kohlenwasserstoffe wie Pentan, Hexan, Heptan, Benzol, Ether wie Diethylether und Tetrahydrofuran.

Als lithiumorganische Verbindung wird bevorzugt n-Butyl-lithium verwendet.

Es wird bei Temperaturen von -100° C bis +50° C und bei Normaldruck gearbeitet.

Setzt man bei Verfahren 12b) als Verbindung der Formel (XV) 2-Nitro-1-[2-(2-methoxyethoxy)-5-pyridyl]-propen-1 ein, läßt sich das Verfahren durch folgendes Formelschema wiedergeben:

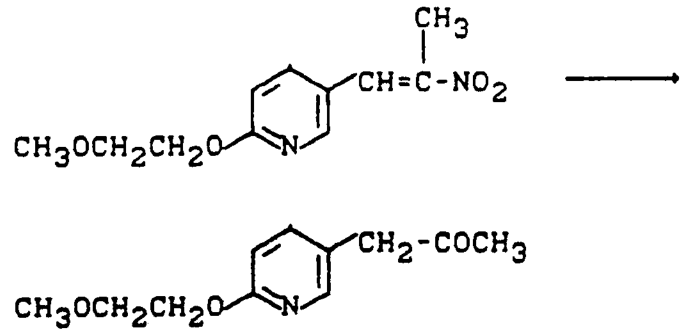

Verbindungen der Formel (XV) sind neu. Ihre Herstellung wird weiter unten beschrieben. Die Substituenten $R^{10}$ und Z besitzen bevorzugt die weiter oben angegebenen bevorzugten Bedeutungen. Im einzelnen seien folgende Verbindungen der Formel (XV) genannt:

2-Nitro-1-[2-(2-ethoxyethoxy)-5-pyridyl]-propen-1
2-Nitro-1-(2-methoxy-5-pyridyl)-propen-1
2-Nitro-1-(2-ethoxy-5-pyridyl)-propen-1

Das Verfahren wird durchgeführt, indem man eine Verbindung der Formel (XV) in Gegenwart einer anorganischen oder organischen Säure reduziert, wobei das intermediär gebildete Oxim direkt zum Keton hydrolysiert wird.

Die Verbindung der Formel (XV) kann auch mit Wasserstoff in Gegenwart eines Katalysators reduziert und anschließend hydrolisiert werden.

Als Reduktionsmittel werden Eisen/Salzsäure oder Zink/Eisessig verwendet.

Die katalytische Reduktion erfolgt vorzugsweise mit Palladium/Kohle in Pyridin, anschließend folgt Hydrolyse mit verdünnten Mineralsäuren wie Schwefelsäure oder Salzsäure.

Die Reaktion wird bei Temperaturen von Raumtemperatur bis zur Siedetemperatur des verwendeten Lösungsmittels durchgeführt.

Es wird bei Normaldruck oder unter erhöhtem Druck gearbeitet.

Setzt man bei Verfahren 14) als Verbindung der Formel (XVII) p-Toluolsulfonsäure-2-methoxyethylester ein, läßt sich das Verfahren durch folgendes Formelschema wiedergeben:

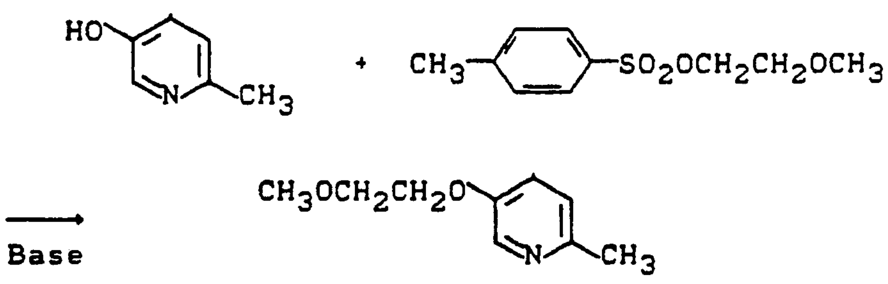


Verbindungen der Formel (XVII) sind bekannt. Im einzelnen seien folgende Verbindungen der Formel (XVII) genannt:

Methansulfonsäure-2-methoxyethylester
Methansulfonsäure-2-ethoxyethylester
p-Toluolsulfonsäure-2-ethoxyethylester

Das Verfahren wird durchgeführt, indem man äquimolare Mengen 5-Hydroxy-2-methylpyridin und Verbindung (XVII) in Gegenwart äquimolarer Menge einer Base in einem Verdünnungsmittel umsetzt.

Als Verdünnungsmittel werden bevorzugt Alkohole wie Methanol oder Ethanol verwendet.

Als Basen werden die entsprechenden Alkalialkoholate wie Natriummethylat und -ethylat verwendet.

Die Reaktion wird bei Raumtemperatur bis Siedetemperatur des verwendeten Lösungsmittels durchgeführt. Es wird bevorzugt bei Normaldruck gearbeitet.

Setzt man bei Verfahren 16) als Verbindung der Formel (XVIII) 2-Ethoxypyridin-5-aldehyd ein, läßt sich das Verfahren durch folgendes Formelschema wiedergeben:

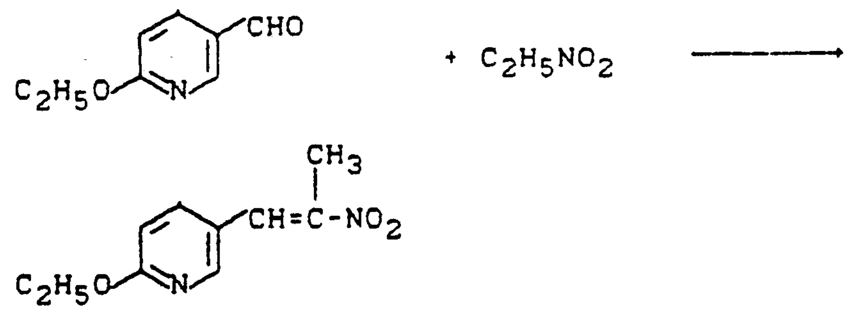

Aldehyde der Formel (XVIII) sind teilweise neu. Die Herstellung der neuen Verbindungen der Formel (XVIII) wird weiter unten (Verfahren 18 a und b) beschrieben. Die Substituenten $R^{10}$ und Z besitzen bevorzugt die weiter oben angegebenen bevorzugten Bedeutungen. Im einzelnen seien folgende Verbindungen der Formel (XVIII) genannt:

2-(2-Methoxyethoxy)pyridin-5-aldehyd

2-(2-Ethoxyethoxy)pyridin-5-aldehyd

Das Verfahren wird durchgeführt, indem man äquivalente Mengen der Verbindung (XVIII) und Nitroethan in einem Verdünnungsmittel unter Zusatz einer Base kondensiert.

Die Reaktion wird bei Temperaturen von 0 °C bis +150 °C durchgeführt.

Es wird bevorzugt bei Normaldruck gearbeitet.

Als Verdünnungsmittel dienen aromatische Kohlenwasserstoffe wie Benzol, Toluol, Xylol, Alkohole wie Methanol, Ethanol, organische Carbonsäuren wie Essigsäure.

Als Basen werden verwendet: Primäre Amine wie Methylamin, Ethylamin, Kombinationen von Hydrochloriden primärer Amine wie Methylaminhydrochlorid, Ethylaminhydrochlorid mit Natriumcarbonat, sekundäre Amine wie Piperidin, tertiäre Amine wie Triethylamin, Ammoniumsalze organischer Carbonsäuren wie Ammoniumacetat.

Setzt man bei Verfahren 18 a als Verbindung der Formel (XIX) 2-Ethoxy-5-pyridylmethanol ein, läßt sich das Verfahren durch folgendes Formelschema darstellen:

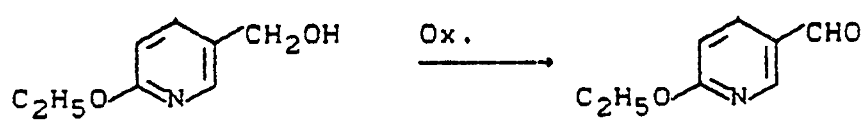


Verbindungen der Formel (XIX) sind teilweise neu. Die Herstellung der neuen Verbindungen der Formel (XIX) wird weiter unten (Verfahren 20) beschrieben. Die Substituenten $R^{10}$ und Z besitzen bevorzugt die weiter oben angegebenen bevorzugten Bedeutungen. Im einzelnen seien folgende Verbindungen der Formel (XIV) genannt:

2-(2-Methoxyethoxy)-5-pyridylmethanol

2-(2-Ethoxyethoxy)-5-pyridylmethanol

Als Oxidationsmittel zur Durchführung des Verfahrens 18a) seien genannt: a) aktiviertes DMSO wie DMSO/Acetanhydrid, DMSO/Thionylchlorid, DMSO/Oxalylchlorid sowie b) Mangandioxid.

Verfahren 18aa) wird durchgeführt, indem man den Alkohol der Formel (XIX) mit 1-1,5 Äquivalenten des Oxidationsmittels umsetzt.

Die Reaktion wird bei Temperaturen von -70 °C bis +25 °C durchgeführt.

Es wird bevorzugt bei Normaldruck gearbeitet.

Als Verdünnungsmittel dienen inerte organ. Lösungsmittel, beispielhaft seien genannt:

Gegebenenfalls chlorierte Kohlenwasserstoffe wie Methylenchlorid, Chloroform, Ether wie Diethylether und Tetrahydrofuran.

Verfahren 18ab) wird durchgeführt, indem man den Alkohol der Formel (XIX) mit überschüssigem Mangandioxid umsetzt.

Die Reaktion wird bei Temperaturen von +20 °C bis +150 °C durchgeführt, es wird bevorzugt bei Normaldruck gearbeitet.

Als Verdünnungsmittel werden verwendet: inerte organische Lösungsmittel, insbesondere aliphatische und aromatische, gegebenenfalls halogenierte Kohlenstoffe wie Pentan, Hexan, Cyclohexan, Benzol, Toluol, Methylenchlorid, Chloroform, Ether wie Diethylether und Tetrahydrofuran.

Verfahren 18 b zur Herstellung der Verbindungen der Formel XVIII wird analog dem von A.S. Dainter et. al., Tetrahedron Letters 25 (1984), S. 5693 - 5696 beschriebenen Verfahrenn durchgeführt.

Setzt man bei Verfahren 20 als Verbindung der Formel (XX) 6-Ethoxynicotinsäure ein, läßt sich das Verfahren durch folgendes Reaktionsschema wiedergeben:

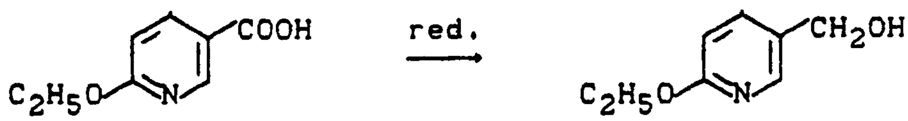


Verbindungen der Formel (XX) sind zum Teil bekannt. Die Herstellung der neuen Verbindungen der Formel (XX) ist weiter unten (Verfahren 22) beschrieben. Die Substituenten $R^{10}$ und Z besitzen bevorzugt die weiter oben angegebenen bevorzugten Bedeutungen. Im einzelnen seien folgende Verbindungen der Formel (XX) genannt:
6-Methoxynicotinsäure,
6-(2-Methoxyethoxy)nicotinsäure,
6-(2-Ethoxyethoxy)nicotinsäure.

Als Reduktionsmittel dienen Borane wie z.B. Diboran, komplexe Metallhydride wie z.B. $LiAlH_4$.

Das Verfahren wird durchgeführt, indem man die Verbindung der Formel (XX) in einem Verdünnungsmittel mit der 1-4 molaren Menge Reduktionsmittel umsetzt.

Die Reaktion wird bei Temperaturen von -50 °C bis +100 °C durchgeführt, es wird bevorzugt bei Normaldruck gearbeitet.

Als Verdünnungsmittel dienen Ether wie Diethylether, Tetrahydrofuran, Dioxan.

Setzt man bei Verfahren 22) als Verbindung der Formel (XXI) 2-Ethoxyethanol ein, läßt sich das Verfahren durch folgendes Formelschema darstellen:

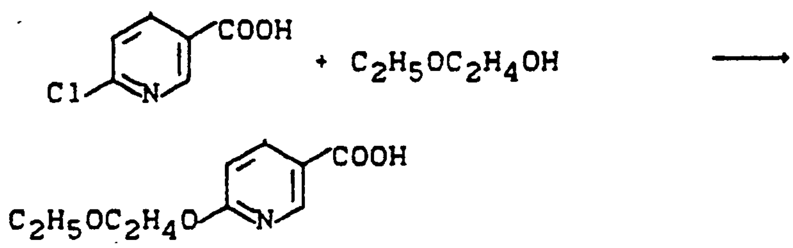

Verbindungen der Formel (XXI) sind bekannt. Die Substituenten $R^{10}$ und Z besitzen bevorzugt die weiter oben angegebenen bevorzugten Bedeutungen. Im einzelnen seien folgende Verbindungen der Formel (XXI) genannt:
Methanol, Ethanol, 2-Methoxyethanol, 2-Ethoxyethanol.

Das Verfahren wird durchgeführt, indem man 6-Chlornicotinsäure mit der 1-5fachen, bevorzugt 1,5-2fachen molaren Menge der Verbindung der Formel (XXI) in Gegenwart der 2-10fachen, bevorzugt 4-7fachen molaren Menge einer Base und 5-25, bevorzugt 10-15 Molprozent eines quartären Ammoniumsalzes in einem Verdünnungsmittel umsetzt. Als Basen werden Alkali- und Erdalkalihydroxide wie z.B. Natriumhydroxid, Kaliumhydroxid, Alkali- und Erdalkalicarbonate wie Natriumcarbonat, Kaliumcarbonat verwendet. Als quartäre Ammoniumsalze werden bevorzugt Tetraalkylammoniumhalogenide, z.B. Tetrabutylammoniumbromid eingesetzt.

Als Verdünnungsmittel werden verwendet: gesättigte und ungesättigte aliphatische und aromatische Kohlenwasserstoffe wie Pentan, Hexan, Heptan, Cyclohexan, Benzol, Toluol, Ether wie Tetrahydrofuran, Dioxan sowie Dimethylformamid und Dimethylsulfoxid.

Die Reaktion wird bei Temperaturen von +20 °C bis +180 °C, bevorzugt bei der Siedetemperatur des verwendeten Verdünnungsmittels, durchgeführt.

Es wird bevorzugt bei Normaldruck gearbeitet.

Setzt man bei Verfahren 24 als Verbindung der Formel XXIII 1-(2-Ethoxy-5-pyridyl)-2-methylpropen-1 ein, läßt sich das Verfahren durch folgendes Reaktionsschema wiedergeben:

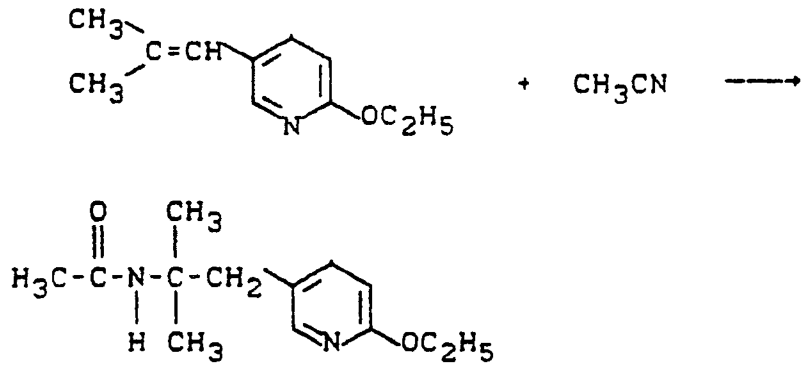

Die Verbindungen der Formel XXIII sind neu. Die Herstellung wird weiter unten (Verfahren 26) beschrieben. Der Substituent $R^4$ besitzt in Formel XXIII bevorzugt die bei den Verbindungen der Formel I angegebenen Bedeutungen. Im einzelnen seien folgende Verbindungen der Formel (XXIII) genannt:

1-[2-(2-Methoxyethoxy)-5-pyridyl]-2-methylpropen-1

1-[2-(2-Ethoxyethoxy)-5-pyridyl]-2-methylpropen-1

Als organische Nitrile zur Durchführung der Reaktion seien bevorzugt genannt: Acetonitril, Propionitril, Benzonitril. Die Reaktion kann auch mit Alkali- oder Erdalkalicyaniden und mit Blausäure durchgeführt werden.

Die Reaktion wird in Gegenwart von Säuren durchgeführt. als solche seien bevorzugt genannt: Schwefelsäure, Perchlorsäure, Phosphorsäure, Polyphosphorsäure, Alkylsulfonsäuren wie Methansulfonsäure, Trifluormethansulfonsäure.

Die Reaktion wird bei Temperaturen von -20 bis +70° C, bevorzugt von -10 bis +50° C, durchgeführt.

Es werden 1 bis 2 Äquivalente der Nitrile bzw. Cyanide und 1 bis 5 Äquivalente Säure pro Mol Verbindung der Formel XXIII eingesetzt. Die Reaktion kann auch in einem Überschuß der Säure, die dann als Verdünnungsmittel dient, durchgeführt werden.

Die Reaktion kann in allen unter den Reaktionsbedingungen inerten organischen Verdünnungsmitteln durchgeführt werden. Hierzu gehören insbesondere aliphatische, aromatische, gegebenenfalls substituierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol, o-Dichlorbenzol, Ether wie Diethyl- oder Dibutylether, Dioxan, Essigsäure, Propionsäure.

Nach Ende der Reaktion wird das Gemisch mit Wasser verdünnt, neutralisiert, von festen Produkten abfiltriert und gegebenenfalls mit nicht vermischbaren Lösungsmitteln extrahiert.

Setzt man bei Verfahren 26 a) als Verbindung der Formel XXIV 2-Methoxymethoxy-5-(1-hydroxy-2-methylpropyl)-pyridin ein, läßt sich das Verfahren durch folgendes Formelschema darstellen:

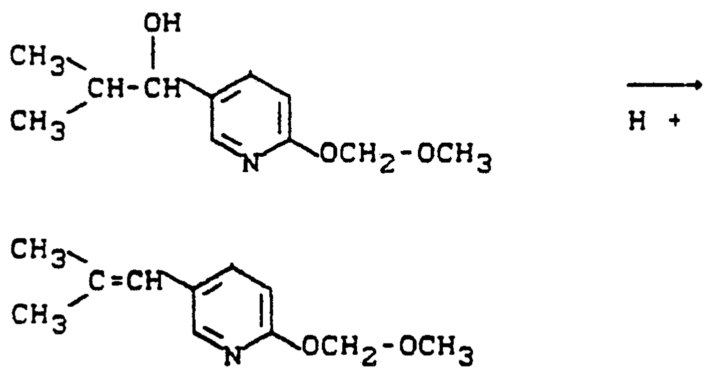


Die Verbindungen der Formel XXIV sind neu. Ihre Herstellung wird weiter unten (Verfahren 18) beschrieben. $R^4$ besitzt die in Formel XXIV, bevorzugt die bei den Verbindungen der Formel I angegebenen Bedeutungen. Im einzelnen seien folgende Verbindungen der Formel XXIV genannt:

2-(2-Methoxyethoxy)-5-(1-hydroxy-2-methylpropyl)pyridin

2-(2-Ethoxyethoxy)-5-(1-hydroxy-2-methylpropyl)pyridin

Die Reaktion wird in Gegenwart von bis zu 30 Vol.-% (bezogen auf die Verbindung der Formel XXIV) einer Protonensäure durchgeführt. Gegenbenenfalls kann auch auf den Säurezusatz verzichtet werden. Als Säuren kommen infrage: Halogenwasserstoffsäuren wie z.B. Salzsäure, Schwefelsäure, Phosphorsäuren, aliphatische und aromatische Sulfonsäuren wie z.B. Methansulfonsäure, p-Toluolsulfonsäure sowie saure Ionenaustauscherharze.

Die Reaktion wird bei 50 bis 250° C, bevorzugt bei 50 bis 180° C und Normaldruck durchgeführt.

Es kann gegebenenfalls in Gegenwart unter den Reaktionsbedingungen inerter Lösungsmittel gearbeitet werden. Als solche seien genannt: aliphatische und aromatische Kohlenwasserstoffe wie Pentan, Hexan Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, außerdem Ester wie Essigsäuremethylester und -ethylester, ferner Nitrile wie z.B. Acetonitril und Propionitril, Benzonitril, darüber hinaus Amide wie z.B. Dimethylformamid, Dimethylacetamid und N-Methylpyrrolidon sowie Tetramethylensulfon und Hexamethylphosphorsäuretriamid.

Nach Beendigung der Reaktion wird das Reaktionsgemisch neutralisiert, von Lösungsmittel abdestilliert und das Produkt isoliert.

Verfahren 26 b wird in 2 Stufen durchgeführt und läßt sich durch folgendes Reaktionsschema beschreiben. Zunächst wird die Verbindung der Formel XXIV durch Reaktion mit einem Halogenierungsmittel in das entsprechende Halogenid überführt und dieses anschließend in Gegenwart einer Base zum vinylsubstituierten Pyridin umgesetzt.

Als Halogenierungsmittel seien genannt: Sulfonylchlorid, Phosgen, Phosphoroxychloride, $PCl_3$, $PCl_5$ sowie die entsprechenden Bromide. Anstelle eines Halogenierungsmittels kann auch ein Sulfonylierungsmittel wie Methansulfonsäurechlorid verwendet werden.

Die Halogenierung bzw. Sulfonylierung wird bei -20 bis +150° C, bevorzugt bei 0 bis 80° C und unter Normaldruck durchgeführt.

Es werden 1 bis 2 Äquivalente Halogenierungsmittel bzw. Sulfonylierungsmittel pro Mol Verbindung der Formel XXIV eingesetzt.

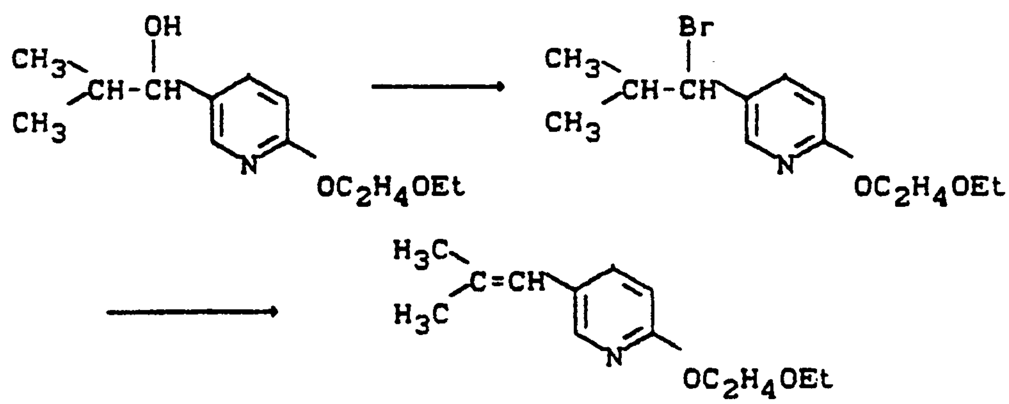

Das Halogenierungsmittel kann im Überschuß als Verdünnungsmittel verwendet werden. Weitere Verdünnungsmittel sind: aliphatische und aromatische Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, außerdem Ester wie Essigsäuremethylester und -ethylester, ferner Nitrile wie z.B. Acetonitril und Propionitril, Benzonitril, darüber hinaus Amide wie z.B. Dimethylformamid, Dimethylacetamid und N-Methylpyrrolidon sowie Tetramethylensulfon und Hexamethylphosphorsäuretriamid.

Die Aufarbeitung erfolgt nach Neutralisieren des Reaktionsgemisches in üblicher Weise.

Für die Eliminierungsreaktion zum Olefin seien genannt: Alkalialkoholate wie Natriummethylat, Kalium, tert.-Butylat, Alkaliamide wie Natriumamid, Lithiumdiisopropylamid, tertiäre Amine wie Ethyldiisopropylamin, Diazabicycloundecen, Diazabicyclononen, Alkalihydroxide wie NaOH und KOH.

Die Eliminierung wird bei 20 bis 200° C, bevorzugt bei 50 bis 150° C und unter Normaldruck durchgeführt.

Es werden 1 bis 1,5 Äquivalente Base pro Mol eingesetzt. Flüssige Basen können auch als Lösungsmittel verwendet werden. Als weitere Verdünnungsmittel kommen praktisch alle inerten organischen

Lösungsmittel in Frage. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, außerdem Ester wie Essigsäure-methylester und -ethylester, ferner Nitrile wie z.B. Acetonitril und Propionitril, Benzonitril, Glutarsäuredinitril, darüber hinaus Amide wie z.B. Dimethylformamid, Dimethylacetamid und N-Methylpyrrolidon sowie Tetramethylensulfon und Hexamethylphosphorsäuretriamid.

Setzt man bei Verfahren 28 als Verbindung der Formel XVIII 2-(2-Ethoxyethoxy)-pyridin-5-carbaldehyd ein, läßt sich das Verfahren durch folgendes Reaktionsschema wiedergeben:

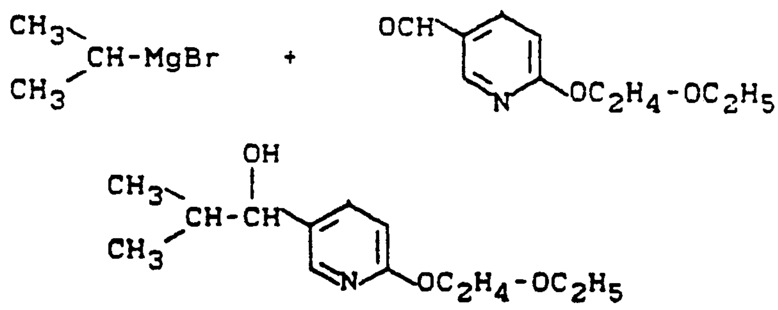

Die Verbindungen der Formel XVIII sind neu. Die Herstellung wird unter Verfahren 18 beschrieben. Es werden bevorzugt die bei Verfahren 16 genannten Verbindungen der Formel XVIII eingesetzt.

Die Grignard-Verbindungen der Formel XXV sind bekannt. Es wird 1 bis 2 Mol Grignard-Verbindung der Formel XXV pro Mol Verbindung der Formel XVIII eingesetzt.

Es wird bei Temperaturen von - 20 bis 100° C, bevorzugt von 0 bis 80° C, gearbeitet.

Als Verdünnungsmittel seien bevorzugt genannt: aliphatische und aromatische Kohlenwasserstoffe wie Pentan, Hexan, Heptan, Cyclohexan, Petrolether, Benzin, Ligroin, Benzol, Toluol, Chlorbenzol und o-Dichlorbenzol, ferner Ether wie Diethyl- und Dibutylether, Glykoldimethylether und Diglykoldimethylether, Tetrahydrofuran und Dioxan, sowie und Hexamethylphosphorsäuretriamid.

Die Aufarbeitung erfolgt nach Beendigung der Reaktion durch Hydrolysieren, Neutralisieren und Extrahieren.

Die Wirkstoffe werden als Leistungsförderer bei Tieren zur Förderung und Beschleunigung des Wachstums, der Milch- und Wollproduktion, sowie zur Verbesserung der Futterverwertung, der Fleischqualität und zur Verschiebung des Fleisch-Fett-Verhältnisses zugunsten von Fleisch eingesetzt. Die Wirkstoffe werden bei Nutz-, Zucht-, Zier- und Hobbytieren verwendet. Sie dienen ferner zur Reduzierung des Fettansatzes bei übergewichtigen Tieren sowie als Mittel zur Behandlung der Adipositas bei Mensch und Tier.

Zu den Nutz- und Zuchttieren zählen Säugetiere wie z.B. Rinder, Schweine, Pferde, Schafe, Ziegen, Kaninchen, Hasen, Damwild, Pelztiere wie Nerze, Chinchilla, Geflügel wie z.B. Hühner, Gänse, Enten, Truthühner, Tauben, Fische wie z.B. Karpfen, Forellen, Lachse, Aale, Schleien, Hechte, Reptilien wie z.B. Schlangen und Krokodile.

Zu den Zier- und Hobbytieren zählen Säugetiere wie Hunde und Katzen, Vögel wie Papageien, Kanarienvögel, Fische wie Zier- und Aquarienfische z.B. Goldfische.

Die Wirkstoffe werden unabhängig vom Geschlecht der Tiere während allen Wachstums- und Leistungsphasen der Tiere eingesetzt.

Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Verabreichung erfolgt oral oder parenteral in dafür geeigneten Formulierungen oder in reiner Form.

Orale Formulierungen sind Pulver, Tabletten, Granulate, Doenche, Boli sowie Futtermittel, Prämixe für Futtermittel, Formulierungen zur Verabreichung über Trinkwasser.

Die oralen Formulierungen enthalten den Wirkstoff in Konzentrationen von 0,01 ppm - 100 %, bevorzugt von 0,01 ppm - 1 %.

Die Wirkstoffe können einmalig verabreicht werden. Die Wirkstoffe können aber auch während der ganzen oder während eines Teils der Wachstums- und Leistungsphase temporär oder kontinuierlich verabreicht werden.

Bei kontinuierlicher Verabreichung kann die Anwendung ein- oder mehrmals täglich in regelmäßigen oder unregelmäßigen Abständen erfolgen.

Die Wirkstoffe können in den Formulierungen allein oder in Mischung mit anderen leistungsfördernden Wirkstoffen, mineralischen Futtermitteln, Spurenelement-Verbindungen, Vitaminen, Stickstoff liefernden Nicht-Protein-Verbindungen, Farbstoffen, Antioxidantien, Aromastoffen, Emulgatoren, Fließhilfsstoffen, Konservierungs- und Preßhilfsstoffen vorliegen.

Andere leistungsfördernde Wirkstoffe sind:
z.B. Antibiotika wie Tylosin und Virginamycin.
Mineralische Futtermittel sind z.B. Dicalciumphosphat, Magnesiumoxid, Natriumchlorid.
Spurenelement-Verbindungen sind z.B. Eisenfumarat, Natriumjodid, Kobaltchlorid, Kupfersulfat, Zinkoxid.
Vitamine sind z.B. Vitamin A, Vitamin $D_3$, Vitamin E, B-Vitamine, Vitamin C.

Stickstoff-liefernde Nicht-Protein-Verbindungen sind z.B. Biuret, Harnstoff.
Farbstoffe sind z.B. Carotinoide wie Citranaxanthin, Zeaxanthin, Capsanthin.
Antioxidantien sind z.B. Ethoxyquin, Butylhydroxy-Toluol.
Aromastoffe sind z.B. Vanillin.
Emulgatoren sind z.B. Ester der Milchsäure, Lecithin.
Fließhilfsstoffe sind z.B. Natriumstearat, Calciumstearat.

Konservierungsstoffe sind z.B. Zitronensäure, Propionsäure.
Preßhilfsstoffe sind z.B. Ligninsulfonate, Celluloseether.

Die Verabreichung der Wirkstoffe kann auch zusammen mit dem Futter und/oder dem Trinkwasser erfolgen.

Zum Futter zählen Einzelfuttermittel pflanzlicher Herkunft wie Heu, Rüben, Getreidenebenprodukte, Einzelfuttermittel tierischer Herkunft wie Fleisch, Fette, Milch produkte, Knochenmehl, Fischprodukte, die Einzelfuttermittel wie Vitamine, Proteine, Aminosäuren z.B. DL-Methionin, Salze wie Kalk und Kochsalz. Zum Futter zählen auch Ergänzungs-, Fertig- und Mischfuttermittel. Diese enthalten Einzelfuttermittel in einer Zusammensetzung, die eine ausgewogene Ernährung hinsichtlich der Energie- und Proteinversorgung sowie der Versorgung mit Vitaminen, Mineralsalzen und Spurenelementen sicherstellt.

Die Konzentration der Wirkstoffe im Futter beträgt normalerweise etwa 0,01-500 ppm, bevorzugt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das 10 ppm erfindungsgemäßen Wirkstoff enthält:
200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g des unten angegebenen Wirkstoff-Prämix ergeben nach sorgfältigem Mischen 1 kg Futter mit 10 ppm Wirkstoffgehalt.

In einem kg Vitamin-Mineral sind enthalten:
600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg $Mn\,SO_4 \times H_2O$, 140 mg $Zn\,SO_4 \times 7\,H_2O$, 100 mg $Fe\,SO_4 \times 7\,H_2O$ und 20 mg $Cu\,SO_4 \times 5\,H_2O$.

2,5 g Wirkstoff-Prämix enthalten z.B. 10 mg Wirkstoff, 1 g DL-Methionin, Rest Sojabohnenmehl.

Beispiel für die Zusammensetzung eines Schweineaufzuchtfutters, das 8 ppm erfindungsgemäßen Wirkstoff enthält:
630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z.B. wie beim Kükenfutter) ergeben nach sorgfältigem Mischen 1 kg Futter mit 8 ppm Wirkstoffgehalt.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere verwendet werden.

Beispiel A

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammenetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist, gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Während des 13-tägigen Versuchs werden Gewichtszunahme und Futterverbrauch bestimmt und die relative Gewichtszunahme im Vergleich zur unbehandelten Kontrolle errechnet.

Es werden die aus der Tabelle ersichtlichen Ergebnisse erhalten:

Tabelle 1

| Ratten Fütterungsversuch | | | |
|---|---|---|---|
| Wirkstoff Beispiel Nr. | Wirkstoff Aufwand ppm | relative Gewichtszunahme % | relative Fettreducktion % |
| 4 | 25 | 25 | 20 |
| 7 | 25 | 13 | 15 |
| 8 | 25 | 44 | 25 |
| 9 | 25 | 30 | 20 |
| 12 | 25 | 10 | 17 |
| | 25 | | |

Beispiele

Allgemeine Vorschrift für Verfahren 2a

Herstellung der Verbindungen der Formel I nach Verfahren 2a

10 mmol der Verbindung der Formel II werden bei 0 °C portionsweise zu einer Lösung von 20 mmol des Amins der Formel III in 15 ml absolutem Ethanol gegeben. Man läßt auf 10-15 °C kommen und rührt bei dieser Temperatur eine Stunde nach. Dann wird wieder auf 0 °C abgekühlt und portionsweise werden 120 mg (10 mmol) Natriumborhydrid zugegeben. Man rührt eine Stunde bei Raumtemperatur nach. Nach Zugabe von 20 ml Wasser wird 30 min gerührt, eingedampft und zwischen Wasser und Essigester verteilt. Die organische Phase wird über Natriumsulfat getrocknet und eingedampft. Der Rückstand wird umkristallisiert oder chromatographiert.

Allgemeine Vorschrift für Verfahren 2b

Herstellung der Verbindungen der Formel I nach Verfahren 2b

0,1 mol der Verbindung der Formel IV und 0,11 mol des Amins der Formel III werden in 200 ml

Methanol über Nacht unter Rückfluß erhitzt. Das Lösungsmittel wird abgezogen und der Rückstand umkristallisiert oder chromatographiert.

Allgemeine Vorschrift für Verfahren 2c

Herstellung der Verbindungen der Formel I nach Verfahren 2c

10 mmol der Verbindung der Formel V werden in 150 ml Ethanol gelöst und 20 mmol des Amins der Formel III werden zugegeben, die Mischung wird 18 Stunden unter Rückfluß erhitzt. Dann wird das Lösungsmittel abgezogen, und der Rückstand wird in 100 ml trockenem Ether aufgenommen. Das unlösliche Aminhydrohalogenid wird abfiltriert, die etherische Lösung mit Wasser gewaschen, über Natriumsulfat getrocknet und eingedampft. Das Rohprodukt wird umkristallisiert oder chromatographiert.

Allgemeine Vorschrift für Verfahren 2d

Herstellung der Verbindungen der Formel I nach Verfahren 2d

10 mmol der Verbindung VI und 10 mmol der Verbindung VII werden unter Zusatz von 4 g Molsieb 4 Å in 40 ml trockenem Ethanol 30 Minuten unter Rückfluß erhitzt. Nach Abkühlen auf 0 °C werden 12 mmol $NaBH_4$ zugegeben, es wird eine Stunde bei 0 °C nachgerührt. Dann stellt man mit verdünnter Salzsäure auf pH 3, zieht das Ethanol ab, nimmt den Rückstand in Wasser auf und wäscht mit Ether. Die Wasserphase wird mit verdünnter Natronlauge alkalisch gestellt und mit Chloroform extrahiert. Nach Trocknen über Natriumsulfat wird eingedampft.

Allgemeine Vorschrift für Verfahren 2e

Herstellung der Verbindungen der Formel I nach Verfahren 2e

Zur Lösung von 10 mmol der Verbindung der Formel VIII in 50 ml Ethanol werden bei 10-15 °C 15 mmol des Amins der Formel III getropft. Man läßt auf Raumtemperatur kommen und rührt noch 15 min nach. Dann verdünnt man mit weiteren 100 ml Ethanol und gibt bei 0-5 °C portionsweise 80 mmol Natriumborhydrid zu. Man läßt auf Raumtemperatur kommen und rührt eine Stunde nach. Dann gibt man bei 10 °C 200 ml Wasser zu, rührt 30 min., dampft das Ethanol ab und extrahiert den Rückstand dreimal mit je 50 ml Dichlormethan. Die vereinigten organischen Phasen werden mit 100 ml Wasser gewaschen, über Natriumsulfat getrocknet und eingedampft.

Allgemeine Vorschrift für Verfahren 2f

Herstellung der Verbindungen der Formel I nach Verfahren 2f

Zu 12,4 ml einer 1 M Lösung von Boran in Tetrahydrofuran werden 2,3 mmol der Verbindung IX in 30 ml absolutem Tetrahydrofuran getropft. Die Mischung wird eine 1 Stunde unter Rückfluß erhitzt, mit Eiswasser verdünnt und mit 50 ml 1N Salzsäure versetzt. Nach Abdampfen des organischen Lösungsmittels wird die saure wäßrige Lösung zweimal mit je 30 ml Ether extrahiert, dann mit gesättigter Natriumcarbonatlösung alkalisch gestellt und dreimal mit je 30 ml Essigester extrahiert. Die vereinigten Extrakte werden über Natriumsulfat getrocknet und eingedampft.

Analog zu den oben angegebenen Verfahren 2a - 2f werden die folgenden Verbindungen hergestellt:

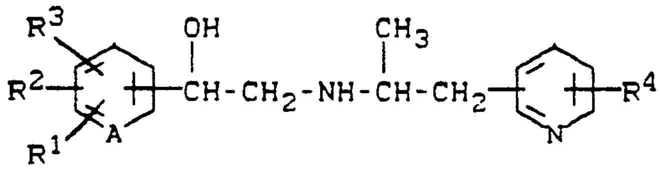

jeweils Diastereomerengemische

| Beispiel Nr. | 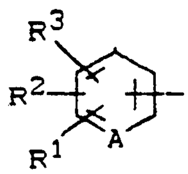 | 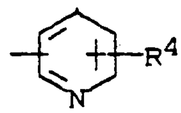 | Ausb. [%] bei Verfahren | $^1$H-NMR ($CDCl_3$, δ [ppm]) |
|---|---|---|---|---|
| 1 | 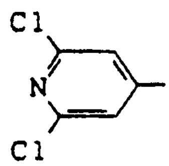 | 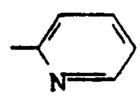 | 60 | 1,1 (dd, 3H); 2,0-3,3 (m, 7H); 4,6 (m, 1H); 7,2 (m, 4H); 7,6 (m, 1H); 8,5 (m, 1H). |
| 2 | 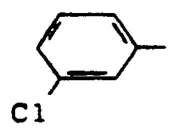 | 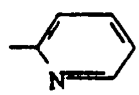 | 86 | 1,1 (dd, 3H); 1,8-3,3 (m, 7H); 4,6 (m, 1H); 7,2 (m, 6H); 7,6 (m, 1H); 8,5 (m, 1H). |

| Beispiel Nr. | 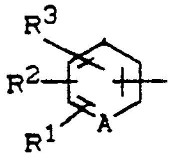 | 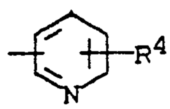 | Ausb. [%] bei Verfahren | $^1$H-NMR ($DCl_3$, δ [ppm]) |
|---|---|---|---|---|
| 3 | 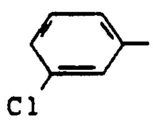 | 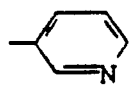 | 65 | 1,2 (dd, 3H); 2,2-3,0 (m, 7H); 4,6 (m, 1H); 7,2 (m, 4H); 7,5 (m, 1H); 8,5 (m, 3H). |
| 4 | 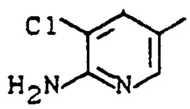 | 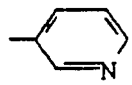 | 61 | 1,2 (dd, 3H); 2,0-3,0 (m, 7H); 4,5 (m, 1H); 4,9 (s, 2H); 7,2 (m, 1H); 7,5 (m, 1H); 7,9 (m, 1H); 8,5 (m, 3H) |
| 5 | 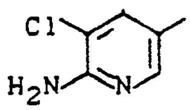 | 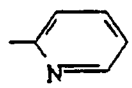 | 61 | 1,2 (dd, 3H); 2,2-3,1 (m, 6H); 3,4 (m, 1H); 4,6 (m, 1H); 5,0 (s, 2H); 7,1 (m, 2H); 7,6 (m, 2H); 7,9 (m, 1H); 8,5 (m, 1H). |
| 6 | 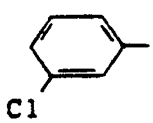 | 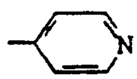 | 71 | 1,1 (dd, 3H); 2,2-3,0 (m, 7H); 4,6 (m, 1H); 7,2 (m, 5H); 8,5 (m, 3H). |
| 7 | 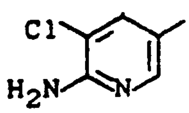 | 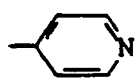 | 73 | 1,1 (dd, 3H); 2,2-3,0 (m, 7H); 4,5 (m, 1H); 4,9 (s, 2H); 7,1 (m, 2H); 7,5 (m, 1H); 7,9 (m, 1H); 8,5 (m, 2H). |

| Beispiel Nr. | 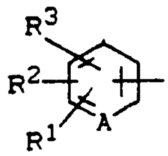 | 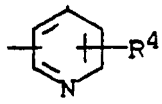 | Ausb. [%] bei Verfahren | $^1H$-NMR ($DCl_3$, δ [ppm]) |
|---|---|---|---|---|
| 8 | 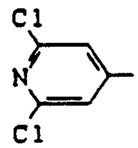 | 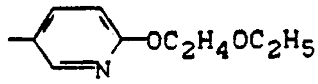 | 99 | 1,1 (dd, 3H);<br>1,3 (t, 3H);<br>1,9-3,1 (m, 7H);<br>3,6 (q, 2H);<br>3,8 (t, 2H);<br>4,4 (t, 2H);<br>4,6 (m, 1H);<br>6,8 (m, 1H);<br>7,2 (m, 2H);<br>7,4 (m, 1H);<br>7,9 (m, 1H). |
| 9 | 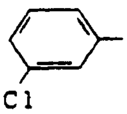 | 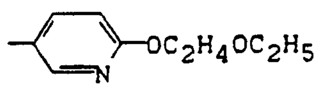 | 97 | 1,1 (dd, 3H);<br>1,3 (t, 3H);<br>2,1-3,0 (m, 7H);<br>3,6 (q, 2H);<br>3,8 (t, 2H);<br>4,4 (t, 2H);<br>4,6 (m, 1H);<br>6,7 (m, 1H);<br>7,2-7,4 (m, 5H);<br>8,0 (m, 1H). |
| 10 | 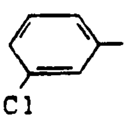 | 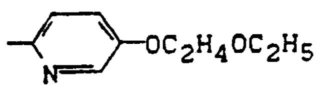 | 80 | 1,1 (dd, 3H);<br>1,2 (t, 3H);<br>2,0-3,0 (m, 7H);<br>3,6 (q, 2H);<br>3,8 (m, 2H);<br>4,1 (m, 2H);<br>4,6 (m, 1H);<br>7,1-7,4 (m, 6H);<br>8,3 (m, 1H). |
| 11 | 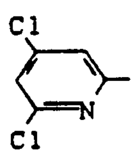 | 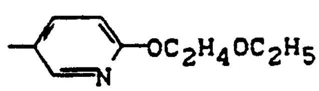 | 97 | 1,1 (dd, 3H);<br>1,2 (t, 3H);<br>1,6-2,9 (m, 6H);<br>3,1 (m, 1H);<br>3,6 (q, 2H);<br>3,8 (t, 2H);<br>4,4 (t, 2H);<br>4,6 (m, 1H);<br>6,7 (m, 1H);<br>7,2 (m, 1H);<br>7,4 (m, 2H);<br>7,9 (m, 1H). |

| Beispiel Nr. | 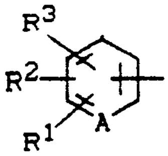 | 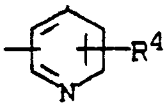 | Ausb. [%] bei Verfahren | $^1H$-NMR ($DCl_3$, δ [ppm]) |
|---|---|---|---|---|
| 12 | 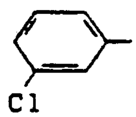 | 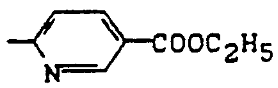 | 64 | 1,1 (dd, 3H); 1,4 (t, 3H); 2,5-3,1 (m, 3H); 4,4 (q, 2H); 4,65 (m, 1H); 7,1-7,4 (m, 5H); 8,2 (m, 1H); 9,1 (m, 1H). |

Beispiel 13

N-[(2-(2-Ethoxi-ethylenoxi)-5-pyridyl)-1,1-dimethylethyl]-1-(3-chlorphenyl)-2-amino-ethanol

0,5 g (2,14 mmol) ω-Brom-3-Chloracetophenon und 2,1 g (4,62 mmol) 2-(2-Ethoxi-ethylenoxi)-5-(2-amino-2-methylpropyl)-pyridin werden in 6 ml trockenem Acetonitril 15 Minuten unter Rückfluß erhitzt. Anschließend wird im Vakuum eingedampft, der Rückstand in 10 ml trockenem Methanol aufgenommen, auf 0 °C gekühlt und 370 mg (9,8 mmol) $NaBH_4$ portionsweise eingetragen. Es wird noch 30 Minuten bei 0 °C gerührt, dann auf 200 ml Wasser gegossen und mit Ether extrahiert. Der Extrakt wird mit $Na_2SO_4$ getrocknet und eingedampft. Zur Reinigung wird über Kieselgel mit Essigester/Methanol chromatographiert.
Ausbeute: 620 mg
Schmelzpunkt: 74 °C

Beispiel 14

650 mg (2,73 mmol) 2-(2-Ethoxi-ethylenoxi)-5-(2-amino-2-methyl-propyl)-pyridin werden mit 510 mg (2,73 mmol) 3-Chlorphenyl-glyoxalhydrat und 2 g 3 Å Molsieb 4 Stunden in 20 ml trockenem Ethanol bei Raumtemperatur gerührt. Anschließend wird mit Eis gekühlt und 300 mg (7,94 mmol) $NaBH_4$ zugegeben und ohne Kühlung eine Stunde gerührt. Zur Aufarbeitung wird filtriert, das Filtrat eingedampft und der Rückstand mit 200 ml Wasser versetzt. Es wird dreimal mit je 70 ml $CH_2Cl_2$ extrahiert, der Extrakt mit $Na_2SO_4$ getrocknet und eingedampft.

Der Rückstand wird durch Chromatographie am Kieselgel mit Essigester/Methanol gereinigt.
Ausbeute: 580 mg
Schmelzpunkt: 74 °C

Analog zu Beispiel 13 und 14 werden hergestellt:

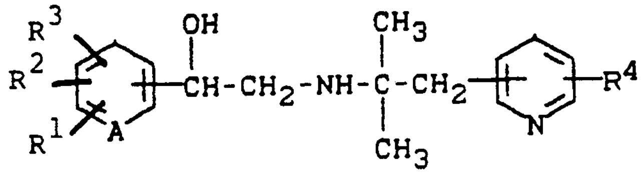

| 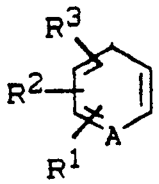 | 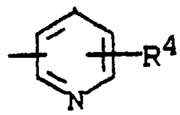 | Ausbeute [%] | $^1$H-NMR ($CDCl_3$, δ[ppm]) |
|---|---|---|---|
| 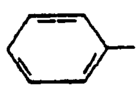 | 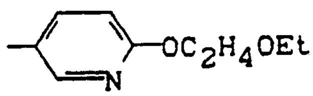 | 27 | 7,9 (d,1H)<br>7,25-7,4 (m,6H)<br>6,72 (d,1H)<br>4,65 (dd,1H)<br>4,45 (t,2H)<br>3,8 (t,2H)<br>3,6 (q,2H)<br>2,97(dd,1H)<br>2,7 (dd,1H)<br>2,61(s,2H)<br>1,25(t,3H)<br>1,08(s,6H) |
| 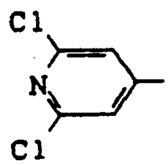 | 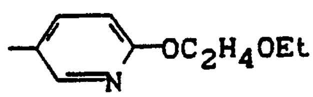 | 26 | 7,9(d,1H), 7,38(dd,1H), 7,3(s,2H), 6,75(d,1H), 4,55(dd,1H), 4,45(t,2H), 3,8(t,2H), 3,6(q,2H), 3,02(dd,1H), 2,6(s,2H), 2,55(dd,1H), 1,25(t,3H), 1,08(s,6H) |

| 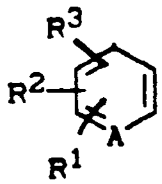 | 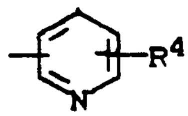 | Ausbeute [%] | $^1$H-NMR ($CDCl_3$, δ[ppm]) |
|---|---|---|---|
| 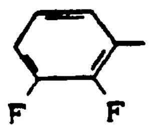 | 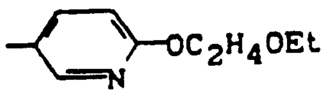 | Ausbeute: 65 % | 7,9 (d,1H)<br>7,25-7,4 (m,2H)<br>7,0 -7,1 (m,2H)<br>6,72 (d,1H)<br>4,95 (dd,1H)<br>4,45 (t,2H)<br>3,8 (t,2H)<br>3,6 (q,2H)<br>3,0 (dd,1H)<br>2,6 (s,2H)<br>2,65(dd,1H)<br>1,25(t,3H)<br>1,08(d,6H) |
| 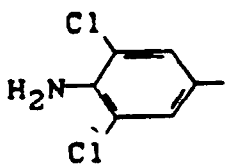 | 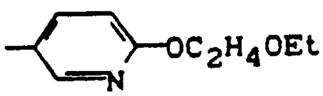 | 57 | 7,9(d,1H), 7,35(dd,1H), 7,2(s,2H), 6,75(d,1H), 4,35-4,5(m,3H), 3,8(t,2H), 3,6(q,2H), 3,9(dd,1H), 2,45-2,65 (m,3H), 1,25(t,3H), 1,05(s,6H)<br><br>Schmelzpunkt 75° C |
| 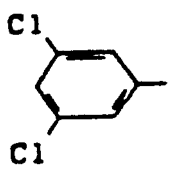 | 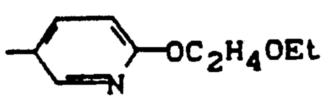 | 25 | Schmelzpunkt 66° C |

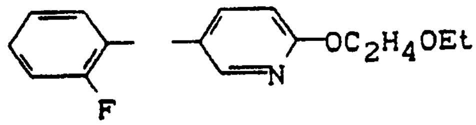 40 Schmelzpunkt 58° C

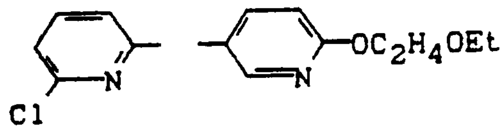 17 7,9(d,1H), 7,67(t,1H), 7,45(d,1H), 7,35(dd,1H), 7,25(d,1H), 6,75(d,1H), 4,2(dd,1H), 4,45(t,2H), 3,28(t,2H), 3,6(q,2H), 3,15(dd,1H), 2,8(dd,1H), 2,5-2,68(m,2H), 1,25(t,3H), 1,07(d,6H)

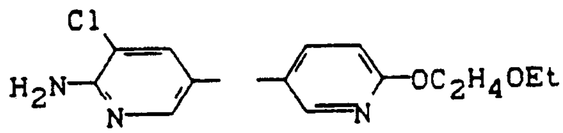 34 Schmelzpunkt 111° C

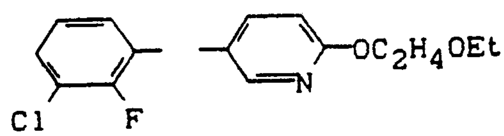 57 Schmelzpunkt 64° C

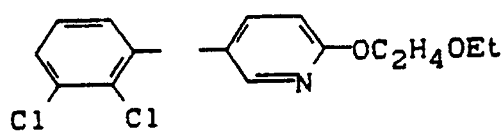 64 Schmelzpunkt 98° C

Beispiel für die Herstellung der Verbindungen VII nach Verfahren 4 b

2 g (7,5 mmol) 2-(2-Ethoxi-ethylenoxi)-5-(2-formylamino-2-methyl-propyl)-pyridin, 1,92 g (21,6 mmol) 45%ige NaOH, 20 ml Methanol und 4 ml Wasser werden 12 Stunden unter Rückfluß erhitzt. Nach Abkühlung wird auf Wasser gegossen und mit Ether extrahiert. Das Extrat wird mit $Na_2SO_4$ getrocknet und eingedampft.

Rückstand: 1,66 g gelbes Öl (93 % der Theorie)

$^1$H-NMR ($CDCl_3$):

7,95 ppm (d, 1H, $H_{arom}$);
7,42 ppm (dd, 1H, $H_{arom}$);
6,75 ppm (d, 1H, $H_{arom}$);
4,46 ppm (t, 2H, $-O-CH_2-$);
3,8 ppm (t,2H, $Ar-CH_2-$);'
3,1 ppm (q, 2H, -OEt);
2,58 ppm (s, 2H, $Ar-CH_2-$);
1,25 ppm (t, 3H, -OEt);
1,1 ppm (s, 6H, $-CH_3$)

Beispiel für die Herstellung der Verbindungen VII bzw. XIII nach Verfahren 12a)

[5-(2-Ethoxyethoxy)-2-pyridyl]-aceton

Zur Lösung von 1 g (5,5 mmol) 5-(2-Ethoxyethoxy)-2-methylpyridin in 20 ml Tetrahydrofuran werden bei 5 °C 2,5 ml einer 2,5 M Lösung von n-Butyllithium in Hexan getropft. Man rührt 10 Minuten bei 0-5 °C nach und gibt dann 530 mg (6 mmol) N,N-Dimethylacetamid, gelöst in wenig Tetrahydrofuran, zu. Nach 90 Minuten Rühren bei Raumtemperatur wird mit verdünnter Salzsäure auf pH 5 gestellt und das Tetrahydrofuran abgezogen. Der Rückstand wird mit Essigester extrahiert, die organische Phase wird mit Natriumsulfat getrocknet und eingedampft.
Ausbeute: 580 mg (47 % d.Th.), Gehalt lt. GC/MS: 75 %.

Beispiel für die Herstellung der Verbindungen VII bzw. XIII nach Verfahren 12b)

[2-(2-Ethoxyethoxy)-5-pyridyl]-aceton

Zu einer siedenden Suspension von 800 mg (3,2 mmol) 1-[2-(2-Ethoxyethoxy)-5-pyridyl]-2-nitro-propen-1 und 710 mg Eisenpulver in 10 ml Methanol werden 6,5 ml konzentrierte Salzsäure getropft. Nach 30 Minuten wird filtriert und mit Wasser verdünnt. Man wäscht mit Ether, stellt auf pH 7 und extrahiert mit Ether. Die organische Phase wird mit 1 %iger wäßriger Natronlauge, dann mit Wasser gewaschen, über Natriumsulfat getrocknet und eingedampft.

Man erhält 420 mg (60 % d.Th.) eines hellgelben Öls.

$^1$H-NMR ($CDCl_3$, $\delta$ [ppm]): 1,2 (t, 3H); 2,2 (s, 3H); 3,55 (q, 2H); 3,7 (s, 2H); 3,8 (m, 2H); 4,5 (m, 2H); 6,8 (d, 1H); 7,4 (dd, 1H); 8,0 (d, 1H).

Beispiel für die Herstellung der Verbindungen XVI nach Verfahren 14)

5-(2-Ethoxyethoxy)-2-methylpyridin

Zur Lösung von 2 g (18,4 mmol) 5-Hydroxy-2-methylpyridin in 20 ml abs. Methanol wird eine Lösung von 994 mg (18,4 mmol) Natriummethylat in 20 ml abs. Methanol getropft. Man rührt 5 Minuten nach und gibt dann die Lösung von 5,83 g (23,9 mmol) p-Toluolsulfonsäure-(2-ethoxy)-ethylester in 15 ml abs. Methanol zu. Die Mischung wird 72 Stunden unter Rückfluß erhitzt. Man dampft zur Trockene ein, nimmt den Rückstand in Essigester auf und wäscht mit gesättigter NaCl-Lösung. Nach Trocknen über Natriumsulfat und Eindampfen erhält man 2,1 g (70 % d.Th.) eines schwach gelben Öls.

$^1$H-NMR ($CDCl_3$, $\delta$ [ppm]): 1,2 (t, 3H); 2,5 (s, 3H); 3,6 (q, 2H); 3,8 (m, 2H); 4,15 (m, 2H); 7,05 (d, 1H); 7,15 (dd, 1H); 8,2 (d, 1H).

Beispiel für die Herstellung der Verbindungen XV nach Verfahren 16)

1-[2-(2-Ethoxyethoxy)-5-pyridyl]-2-nitro-propen-1

Eine Mischung aus 8,3 g (42,6 mmol) 2-(2-Ethoxyethoxy)-pyridin-5-aldehyd, 5,53 g (73,7 mmol) Nitroethan und 3,83 g (49,7 mmol) Ammoniumacetat in 50 ml Eisessig wird 7 Stunden unter Rückfluß erhitzt. Dann wird unter Eiskühlung mit Wasser verdünnt und das Produkt abgesaugt. Ausbeute: 4,8 g (45 % d.Th.), Fp. 75 °C.

Beispiel für die Herstellung der Verbindungen XVIII nach Verfahren 18 a)

2-(2-Ethoxyethoxy)-pyridin-5-aldehyd

Eine siedende Lösung von 8 g (40,6 mmol) [2-(2-Ethoxyethoxy)-5-pyridyl]-methanol in 200 ml Toluol

wird innerhalb von zwei Stunden portionsweise mit insgesamt 16 g Mangandioxid versetzt. Dann wird heiß filtriert und eingedampft. Hellgelbes Öl, Ausb. 7,1 g (90 % d.Th.).

$^1$H-NMR ($CDCl_3$; δ [ppm]): 1,2 (t, 3H); 3,6 (q, 2H); 3,8 (t, 2H); 4,6 (t, 2H); 6,9 (d, 1H); 8,1 (dd, 1H); 8,6 (d, 1H); 10,0 (s, 1H).

<u>Beispiel für die Herstellung der Verbindungen der Formel XVIII nach Verfahren 18 b</u>

2-(2-Ethoxi-ethylenoxi)-5-formyl-pyridin

1,3 g (32,5 mmol) gepulverte NaOH werden in 10 ml (0,1 mol) 2-Ethoxiethanol gelöst, dann bei einer Temperatur von 70 °C innerhalb von 30 Minuten 2 g (10 mmol) 3-Trichlormethylpyridin zugetropft.

Es wird noch 2 Stunden bei 80 °C gerührt, anschließend auf Raumtemperatur gekühlt und abgesaugt. Das Filtrat wird eingedampft und zum Rückstand 20 ml Wasser zugegeben. Die Mischung wird mit 2N-Salzsäure auf pH 3 gestellt und noch zwei Stunden bei Raumtemperatur gerührt. Danach werden 100 ml $CH_2Cl_2$ zugegeben und gut durchgerührt. Die organische Phase wird abgetrennt, mit Wasser gewschen, mit $Na_2SO_4$ getrocknet und eingedampft. Der Rückstand wird an der Ölpumpe im Kugelrohr destilliert.
Ausbeute: 970 mg gelbes Öl, Gehalt: 85 % (GC/MS)

$^1$H-NMR ($CDCl_3$; δ [ppm]):
9,6 (d, 1H, J = 2,3 Hz$H_{arom}$),
8,1 (dd, 1H, $J_1$ = 2,3 Hz);
6,9 (d, 1H, $J_1$ = 8,7 Hz);
4,6 (t, 2H, -O-$CH_2$-);
3,85 (t, 2H, -O-$CH_2$-);
3,6 (q, 2H, -O-Et);
1,25 (t, 3H, -O-Et)

<u>Beispiel für die Herstellung der Verbindungen XIX nach Verfahren 20)</u>

[2-(2-Ethoxyethoxy)-5-pyridyl]-methanol

Zur Lösung von 2,11 g (10 mmol) 6-(2-Ethoxyethoxy)-nicotinsäure in 40 ml abs. Tetrahydrofuran werden bei 0 °C 40 ml einer 1 M Lösung von Boran in Tetrahydrofuran getropft. Man rührt drei Stunden bei Raumtemperatur nach, dann wird unter Kühlung mit konzentrierter Salzsäure angesäuert, noch 30 Minuten nachgerührt und zur Trockene eingedampft. Der Rückstand wird zwischen Essigester und gesättigter Natriumcarbonatlösung verteilt, die Wasserphase nochmals mit Essigester extrahiert. Die vereinigten organischen Phasen werden mit gesättigter NaCl-Lösung gewaschen, über Natriumsulfat getrocknet und eingedampft. Der Rückstand kristallisiert beim Verreiben mit Pentan.
Farblose Kristalle, Ausbeute: 1,6 g (81 % d.Th.), Fp.: 48 °C.

<u>Beispiel für die Herstellung der Verbindungen XX nach Verfahren 22)</u>

6-(2-Ethoxyethoxy)-nicotinsäure

Eine Suspension von 13,2 g (84 mmol) 6-Chlornicotinsäure, 21,1 g (0,53 mol) gepulvertem Natriumhydroxid, 9,5 g (0,106 mol) 2-Ethoxyethanol und 2,64 g (8,2 mmol) Tetrabutylammoniumbromid in 350 ml abs. Toluol wird 15 Stunden unter Rückfluß erhitzt. Man dampft zur Trockene ein und versetzt den Rückstand unter Kühlung mit soviel 10 %iger Salzsäure, daß die Mischung pH 4 hat. Die ausgefallene Säure wird abgesaugt.
Ausbeute: 13,6 g (77 % d.Th.); Fp.: 125 °C.

<u>Beispiel für die Herstellung der Verbindungen XXII gemäß Verfahren 24</u>

2-(2-Ethoxi-ethylenoxi)-5-(2-formylamino-2-methylpropyl)-pyridin

933 mg (18 mmol) NaCN werden vorsichtig bei einer Temperatur von 0 °C in 5,5 ml 96%ige Schwefelsäure eingetragen. Anschließend werden 2 g (7,9 mmol) 2-(2-Ethoxiethylenoxi)-5-(2,2-dimethylvinyl)pyridin bei 0 °C zugetropft. Nach beendeter Zugabe wird mit einem Wasserbad auf 20 °C erwärmt und 15 Minuten gerührt. Zur Aufarbeitung wird vorsichtig in $NaHCO_3$-Lösung eingetropft und zweimal mit je 100 ml Dichlormethan extrahiert. Es wird über $Na_2SO_4$ getrocknet und eingedampft. Der Rückstand wird durch Chromatographie an Kieselgel gereinigt.
Laufmittel: Essigester
Ausbeute: 1,71 g gelbes Öl (81 % der Theorie)
$^1$H-NMR (DMSO-$D_6$) $\delta$ [ppm]):
7,5 (d, 1H, Formyl
7,88 (d, 1H, $H_{arom}$);
7,48 (dd, 1H, $H_{arom}$);
6,75 (d, 1H, $H_{arom}$);
4,32 (t, 2H, -O-$CH_2$-);
3,17 (t, 2H, -O-$CH_2$-);
3,49 (q, 2H, -O-Et);
2,92 (s, 2H, Ar-$CH_2$-);
1,2 (s, 6H, -$CH_3$);
1,12 (t, 3H, -O-Et)

2-(2-Ethoxi-ethylenoxi)-5-(2-acetamino-2-methylpropyl)pyridin

Eine Mischung aus 1 g (4,52 mmol), 2-(2-Ethoxy-ethylenoxi)-5-(2,2-dimethylvinyl)-pyridin und 0,48 ml (9,2 mmol) Acetonitril wird bei Raumtemperatur langsam unter Rühren zu 2,5 ml 96%iger Schwefelsäure getropft. Es wird noch 30 Minuten nachgerührt, dann die Mischung vorsichtig in $NaHCO_3$-Lösung eingetropft. Es wird mit $CH_2Cl_2$ extrahiert, der Extrakt mit $Na_2SO_4$ getrocknet und eingedampft.
Rückstand: 0,9 g
Gehalt: 95 % (GC/MS)
Schmelzpunkt: 87 °C

<u>Beispiel für die Herstellung der Verbindungen XXIII gemäß Verfahren 26 a</u>

2-(2-Ethoxi-ethylenoxi)-5-(2,2-dimethylvinyl)-pyridin

20 g (Gehalt 92 %, 7,7 mmol) 2-(2-Ethoxi-ethylenoxi)-5-(1-hydroxi-2-methyl-propyl)-pyridin, 2 g (10,6 mmol) p-Toluolsulfonsäurehydrat und 200 ml Toluol werden 6 Stunden unter Rückfluß erhitzt. Nach dem Abkühlen wird mit gesättigter $NaHCO_3$-Lösung gewaschen, mit $Na_2SO_4$ getrocknet und eingedampft. Der Rückstand wird durch Säulenchromatographie am Kieselgel mit $CH_2Cl_2$/Essigester als Laufmittel gereinigt.
Ausbeute: 3,84 g (22 % der Theorie) gelbes Öl
$^1$H-NMR ($CDCl_3$) $\delta$ [ppm]):
8,0 (d, 1H, $H_{arom}$);
7,45 (dd, 1H, $H_{arom}$);
6,75 (d, 1H, $H_{arom}$);
6,1 (s, 1H, $H_{vinyl}$);
4,5 (t, 2H, -O-$CH_2$-);
3,8 (t, 2H, -O-$CH_2$-);
3,6 (q, 2H, -O-Et);
1,9 (s, 3H, $CH_3$);
1,25 (t, 3H, -O-Et)

<u>Beispiele zur Herstellung der Verbindungen XXIII nach Verfahren 26 b</u>

2-(2-Ethoxi-ethylenoxi)-5-(1-chlor-2-methyl-propyl)pyridin

23 g (Gehalt 86 %, 78 mmol) 2-(2-Ethoxi-ethylenoxi-5-(1-hydroxi-2-methyl-propyl)-pyridin werden zu 220 ml trockenem $CHCl_3$ gegeben und anschließend unter Eiskühlung 10,1 ml (0,14 mol) Thionylchlorid zugetropft. Nach beendeter Zugabe wird noch eine Stunde bei Raumtemperatur gerührt, dann unter Rühren in $NaHCO_3$-Lösung eingetropft. Die organische Phase wird abgetrennt, die wäßrige Phase mit $CHCl_3$ extrahiert. Die vereinigten organischen Phasen werden mit $Na_2SO_4$ getrocknet und eingedampft.
Ausbeute: 23,8 g
Gehalt: 86 %, (GC/MS) gelbes Öl $^1$H-NMR ($CDCl_3$) $\delta$ [ppm]):
8,03 (d, 1H, $H_{arom}$);
7,6 (dd, 1H, $H_{arom}$);
6,8 (d, 1H, $H_{arom}$);
4,6 (d, 1H, -CH-Cl);
4,5 (t, 2H, -O-$CH_2$-);
3,8 (t, 2H, -O-$CH_2$-);
3,6 (q, 2H, -O-Et);
2,2 (m, 1H, i-Propyl);
1,25 (t, 3H, -OEt);
1,1 (d, 3H, i-Propyl);
0,88 (d, 3H, i-Propyl)

28 g (Gehalt 87 %, 95 mmol) 2-(2-Ethoxi-ethylenoxi-5-(1-chlor-2-methyl-propyl)-pyridin werden zu 140 ml Diazabicycloundecen gegeben und eine Stunde unter Rückfluß erhitzt. Nach Abkühlung wird auf $NaHCO_3$-Lösung gegeben und mit Ether extrahiert. Der Extrakt wird zweimal mit $NaHCO_3$-Lösung gewaschen, mit $Na_2SO_4$ getrocknet und eingedampft.
Rückstand: 21,2 g gelbes Öl
Gehalt: 87 % (GC/MS)

<u>Beispiel zur Herstellung der Verbindungen XXIV nach Verfahren 28</u>

2-(Ethoxi-ethylenoxi)-5-(1-hydroxi-2-methylpropyl)pyridin

6,5 g (0,271 g) Magnesiumspäne werden mit trockenem Ether übergossen und die Reaktion durch einige Tropfen 2-Brompropan gestartet.

Dann werden 25,2 ml (0,271 mol) 2-Brompropan, gelöst in 280 ml trockenem Ether innerhalb von 60 Minuten zugetropft und noch 30 Minuten nachgerührt. Anschließend werden 27 g (Gehalt 85 %, 0,118 mmol) 2-(2-Ethoxiethylenoxi)-pyridin-5-carbaldehyd, gelöst in 130 ml trockenem Ether zugetropft und eine Stunde unter Rückfluß erhitzt. Zur Aufarbeitung wird abgekühlt, auf 2 l gesättigte $NH_4Cl$-Lösung gegossen, die organische Phase abgetrennt, die wäßrige Phase mit Ether extrahiert und nach Trocknung mit $Na_2SO_4$ eingedampft.
Rückstand: 30 g gelbes Öl, Gehalt: 92 % (GC/MS)
$^1$H-NMR ($CDCl_3$) $\delta$ [ppm]):
8,0 (d, 1H, $H_{arom}$);
7,55 (dd, 1H, $H_{arom}$);
6,8 (d, 1H, $H_{arom}$);
4,45 (t, 2H, -O-$CH_2$);
4,3 (d, 1H, -CH-OH);
3,25 (t, 2H, -O-$CH_2$-);
3,1 (q, 2H, -O-Et);
1,9 (m, 1H, i-Propyl);
1,25 (t, 3H, -OEt);
1,0 (d, 3H, i-Propyl);
0,75 (d, 3H, i-Propyl)

**Ansprüche**

1. Neue Aryl- und Heteroarylethanol-pyridylalkylamine der Formel I

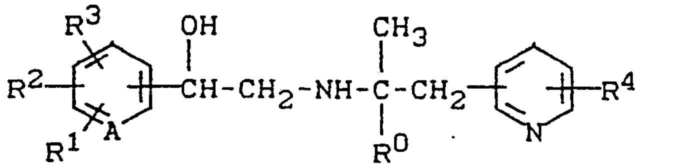

(I)

in welcher
A für =CH- oder =N- steht,
$R^0$ für Wasserstoff oder Methyl steht,
$R^1$ für Wasserstoff, Hydroxy, Halogen, Cyano, Alkyl, Halogenalkyl, Hydroxyalkyl, Alkoxycarbonyl, Aminocarbonyl, Mono- und Dialkylaminocarbonyl, Alkoxy, Halogenalkoxy, Halogenalkylthio, $NHSO_2$-Alkyl steht,
$R^2$ für Wasserstoff, Hydroxy, Alkoxy oder den Rest $-NR^5R^6$ steht,
$R^3$ für die bei $R^1$ angegebenen Reste steht,
$R^4$ für Wasserstoff, $C_1$-$C_{10}$-Alkyl, welches gegebenenfalls durch Hydroxy, Halogen, Alkoxy, Acyloxy oder den Rest $-NR^7R^8$ substituiert ist, sowie für den Rest $COR^9$ oder den Rest $-O-Z-R^{10}$ steht,
Z für $C_1$-$C_{10}$-Alkylen, -Alkenylen oder -Alkinylen steht,
$R^5$ für Wasserstoff oder Alkyl steht,
$R^6$ für Wasserstoff, Alkyl, Halogenalkyl oder Acyl steht, wobei $R^5$ und $R^6$ gemeinsam mit dem angrenzenden N-Atom einen gesättigten oder ungesättigten heterocyclischen 4-, 5- oder 6-Ring bilden können,
$R^7$ für Wasserstoff, gegebenenfalls substituiertes Alkyl, gegebenenfalls substituiertes Aryl steht,
$R^8$ für die bei $R^7$ angegebenen Reste steht,
$R^9$ für Hydroxy, Alkoxy oder den Rest $-NR^7R^8$ steht,
$R^{10}$ für Hydroxy, Alkoxy, Acyloxy, gegebenenfalls substituiertes Aryloxy oder Aralkyloxy steht,
wobei im Pryridylring der Formel I der Substituent $R^4$ und die Alkylaminogruppe in p-Stellung zueinander stehen
sowie ihre physiologisch verträglichen Salze und, wenn A für Stickstoff steht, ihre N-Oxide.

2. Verfahren zur Herstellung der Verbindungen der Formel I

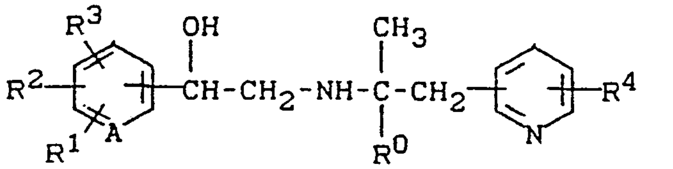

(I)

in welcher
A für =CH- oder =N- steht,
$R^0$ für Wasserstoff oder Methyl steht,
$R^1$ für Wasserstoff, Alkyl, Halogen, Halogenalkyl, Hydroxy, Hydroxyalkyl, Cyano, Alkoxycarbonyl, Aminocarbonyl, Mono- und Dialkylaminocarbonyl, Alkoxy, Halogenalkoxy, Halogenalkylthio, $NHSO_2$-Alkyl steht,
$R^2$ für Wasserstoff, Hydroxy, Alkoxy oder den Rest-$NR^5R^6$ steht,
$R^3$ für die bei $R^1$ angegebenen Reste steht,
$R^4$ für Wasserstoff, $C_1$-$C_{10}$-Alkyl, welches gegebenenfalls durch Hydroxy, Halogen, Alkoxy, Acyloxy oder den Rest $-NR^7R^8$ substituiert ist, sowie für den Rest $COR^9$ oder den Rest $-O-Z-R^{10}$ steht,
Z für $C_1$-$C_{10}$-Alkylen, -Alkenylen oder -Alkinylen steht,
$R^5$ für Wasserstoff oder Alkyl steht,
$R^6$ für Wasserstoff, Alkyl, Halogenalkyl oder Acyl steht, wobei, $R^5$ und $R^6$ gemeinsam mit dem angrenzenden N-Atom einen gesättigten oder ungesättigten heterocyclischen 4-,5- oder 6-Ring bilden können,
$R^7$ für Wasserstoff, gegebenenfalls substituiertes Alkyl, gegebenenfalls substituiertes Aryl steht,
$R^8$ für die bei $R^7$ angegebenen Reste steht,
$R^9$ für Hydroxy, Alkoxy oder den Rest $-NR^7R^8$ steht,
$R^{10}$ für Hydroxy, Alkoxy, Acyloxy, gegebenenfalls substituiertes Aryloxy oder Aralkyloxy steht,
wobei im Pyridylring der Formel I der Substituent $R^4$ und die Alkylaminogruppe in p-Stellung zueinander stehen,

a) indem man Halogenmethylketone der Formel II

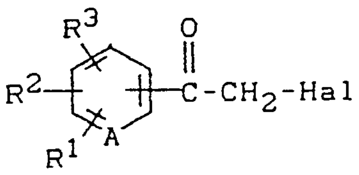

(II)

in welcher
$R^1$ bis $R^3$ und A die oben angegebenen Bedeutung haben und Hal für Halogen steht, mit Aminen der Formel (III)

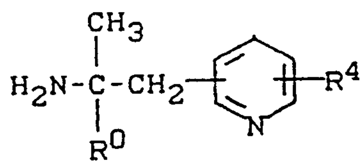

(III)

in welcher
$R^0$ und $R^4$ die oben angegebene Bedeutung haben und $R^4$ in p-Stellung zum Alkylaminrest steht, umsetzt und anschließend die Carbonylgruppe reduziert, oder

b) indem man Epoxide der Formel IV

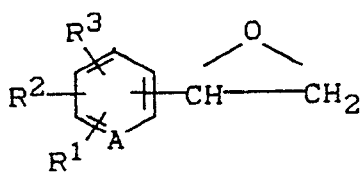

(IV)

in welcher
$R^1$ bis $R^3$ und A die oben angebenene Bedeutung haben, mit Aminen der Formel III

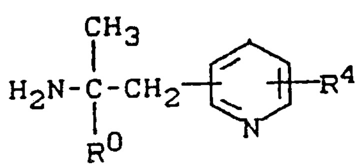

(III)

in which
$R^0$ und $R^4$ die oben angegebene Bedeutung haben und $R^4$ in p-Stellung zum Alkylaminrest steht, umsetzt, oder

c) indem man ß-Halogenethylverbindungen der Formel V

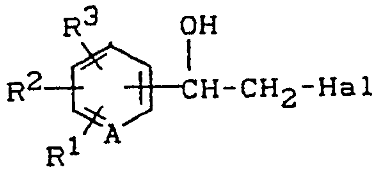

(V)

in welcher
$R^1$ bis $R^3$ und A die oben angegebene Bedeutung haben und Hal für Halogen steht, mit Aminen der Formel III

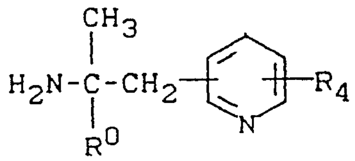

(III)

in welcher
$R^0$ und $R^4$ die oben angegebene Bedeutung haben und $R^4$ in p-Stellung zum Alkylaminrest steht, umsetzt, oder

d) indem man für den Fall, daß $R^0$ für Wasserstoff steht, Verbindungen der Formel VI

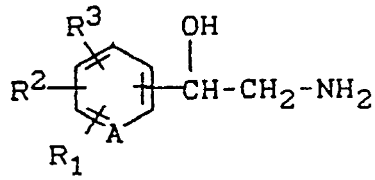

(VI)

in welcher
$R^1$ bis $R^3$ und A die oben angegebene Bedeutung haben, mit Ketonen der Formel VII

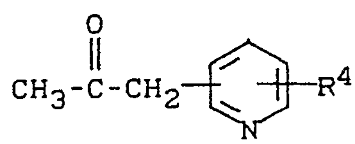

(VII)

in welcher
$R^4$ die oben angegebene Bedeutung hat und in p-Stellung zum Acetonylrest steht, unter reduzierenden Bedingungen umsetzt, oder

e) indem man Verbindungen der Formel VIII

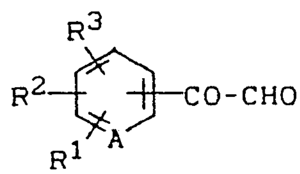

(VIII)

in welcher
$R^1$ bis $R^3$ und A die oben angegebenen Bedeutung haben, mit Aminen der Formel III

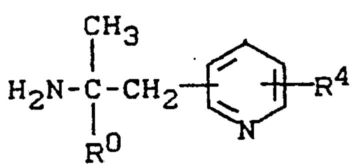

(III)

in welcher
$R^0$ und $R^4$ die oben angebenene Bedeutung haben und $R^4$ in p-Stellung zum Alkylaminrest steht, unter reduzierenden Bedingungen umsetzt, oder

f) indem man Verbindungen der Formel IX

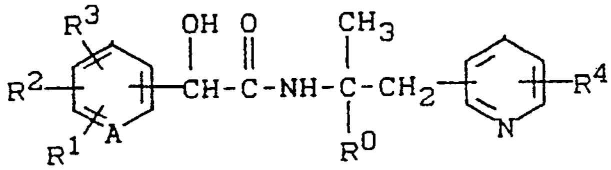

(IX)

in welcher
A und $R^0$ bis $R^4$ die oben angegebene Bedeutung haben, und $R^4$ und der andere Substituent im Pyridinring der Formel IX in p-Stellung zueinander stehen,
reduziert.

3. Neue Verbindungen der Formel III

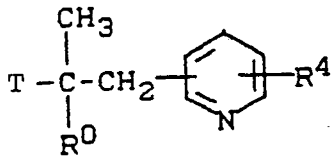

(III)

in welcher
$R^0$ und $R^4$ die bei den Verbindungen der Formel I angegebenen Bedeutungen haben, $R^4$ aber nicht für Wasserstoff steht, und die Substituenten am Pyridinring in p-Postion zueinander stehen,
T für -NC oder -$NH_2$ steht.

4. Neue Verbindungen der Formel IX

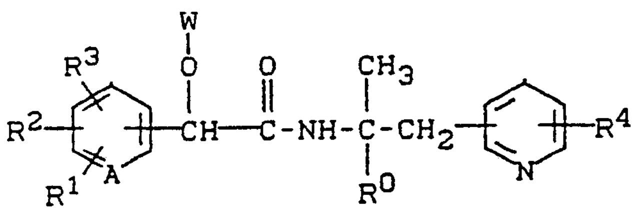

(IX)

in welcher
A und $R^0$ bis $R^4$ die bei den Verbindungen der Formel I angegebenen Bedeutungen haben, wobei $R^4$ und die Alkylaminseitenkette im Pyridinring der Formel (IX) in p-Position zueinander stehen und
W für OH oder -O-$COCH_3$ steht.

5. Neue Verbindungen der Formel XIII

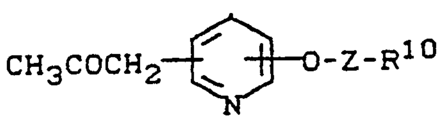

(XIII)

in welcher
$R^{10}$ und Z die in Anspruch 1 angegebene Bedeutung haben und die Substituenten am Pyridinring der Formel (XIII) in p-Position zueinander stehen.

6. Neue Verbindungen der Formel XVI

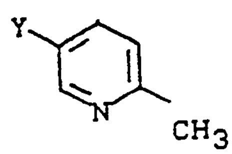

(XVI)

in welcher
Y für -O-Z-$R^{10}$, ausgenommen 2-Methoxyethoxy oder 2-Ethoxyethoxy, steht und
Z und $R^{10}$ die in Anspruch 1 angegebene Bedeutung haben.

7. Neue Verbindungen der Formel XV

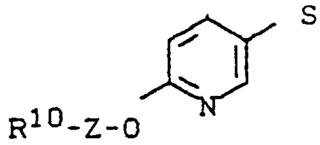

(XV)

in welcher
$R^{10}$ und Z die in Anspruch 1 angegebene Bedeutung haben,
wobei
$R^{10}$-Z- nicht für Methyl steht und
S für

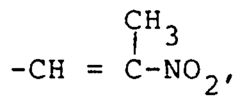

-CHO, $-CH_2-OH$, COOH steht.

8. Neue Verbindungen der Formel (XXII)

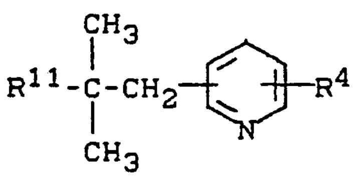

(XXII)

in welcher
$R^4$ die bei den Verbindungen der Formel (I) in Anspruch 1 angegebenen Bedeutungen hat und die Substituenten am Pyridinring der Formel (XXII) in p-Position zueinander stehen,
$R^{11}$ für die Reste -NHCHO und
$-NH\overset{O}{\overset{\|}{C}}-CH_3$ steht.

9. Neue Verbindungen der Formel (XXIII)

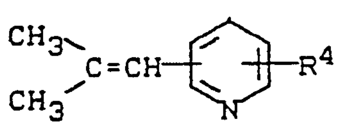

(XXIII)

in welcher
$R^4$ die bei den Verbindungen der Formel (I) in Anspruch 1 angegebene Bedeutung hat und die Substituenten am Pyridinring der Formel (XXIII) in p-Position zu einander stehen.

10. Neue Verbindungen der Formel (XXIII)

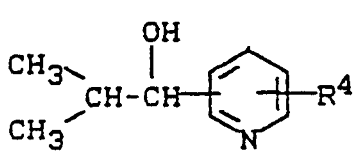

(XXIV)

in welcher
$R^4$ die bei den Verbindungen der Formel (I) in Anspruch 1 angegegebene Bedeutung hat und die Substituenten am Pyridinring der Formel (XXIII) in p-Position zu einander stehen.

11. Mittel zur Leistungsförderung von Tieren sowie zur Bekämpfung der Adipositas bei Mensch und Tier, gekennzeichnet durch einen Gehalt an Aryl- und Heteroarylethanolpyridylalkylaminen der Formel I gemäß Anspruch 1.

12. Tierfutter, Trinkwasser für Tiere, Zusätze für Tierfutter und Trinkwasser für Tiere, gekennzeichnet durch einen Gehalt an Aryl- und Heteroarylethanolpyridylalkylaminen der Formel I gemäß Anspruch 1.

13. Verwendung von Aryl- und Heteroarylethanolpyridylalkylaminen der Formel I gemäß Anspruch 1 zur Leistungsförderung von Tieren sowie zur Behandlung der Adipositas bei Mensch und Tier.